# EUROPEAN PATENT APPLICATION

(11) **EP 4 570 818 A1**
(43) Date of publication of application: **18.06.2025**
(21) Application number: 23852039.9
(22) Date of filing: 26.09.2023
(51) Int. Cl.: C07K 14/47, C07K 1/02, C07K 16/18, A61K 38/17, A61K 39/00, A61K 39/395, A61P 25/28

(54) **HIGHLY TOXIC AMYLOID OLIGOMER AND USE THEREOF**

(30) Priority: 09.08.2022 CN 202210949481
(71) Applicant: Shen Zhen Wisdom Biopharm Co., Ltd., Shenzhen, Guangdong 518067 (CN)
(72) Inventor: LIU, Ruitian, Shenzhen, Guangdong 518067 (CN); HUANG, Yaru, Shenzhen, Guangdong 518067 (CN); XIE, Xixiu, Shenzhen, Guangdong 518067 (CN); YU, Xiaolin, Shenzhen, Guangdong 518067 (CN)
(74) Representative: Høiberg P/S
(86) International application number: PCT/CN2023/121346
(87) International publication number: WO 2024/032821

(57) **Abstract**

Provided is a new and highly toxic amyloid oligomer Aβo*3F. The Aβo*3F is specifically bound by a 3F antibody, is the most important toxic ingredient in a mixture of Aβ oligomers, and has a strong pathogenic effect. Further provided is a binding agent that specifically binds to Aβo*3F, a composition containing Aβo*3F, and a use of Aβo*3F as a target in the prevention and/or treatment and/or diagnosis of MCI and/or AD in subjects.

## Description

### Technical Field

The present invention relates to novel highly toxic amyloid oligomers and uses thereof. Specifically, the present invention relates to novel isolated highly toxic amyloid oligomer Aβo*3F and preparation method thereof, an immunogenic composition, a vaccine containing the same, an antibody against the same, and uses thereof.

### Background

Alzheimer's disease (AD), which is also called senile dementia, is a chronic neurodegenerative disease. There are about 50 million AD patients worldwide. So far, there is no specific effective therapeutic drug or means, which has brought a heavy burden to mankind. AD has a long development course. At first, patients show no substantial clinical symptoms, and gradually develop memory loss and personality and behavior changes. In the later stage, the neurons in AD patients' brain die extensively, the brain shrinks significantly, and the therapeutic drugs are difficult to take effect. Clinical trials in recent years have shown that early intervention for AD patients with early detection and early warning can delay the development of AD. In order to achieve early treatment and intervention of AD patients, they should be identified and diagnosed in a timely and accurate manner first, however, there is currently no ideal early diagnosis method and technology for clinical application. Studies have shown that changes in amyloid β (Aβ) protein that induce the development of AD have already appeared 15 to 20 years before the onset of clinical symptoms.

The pathological characteristics of AD are senile plaques formed by Aβ aggregation and neurofibrillary tangles formed by tau protein aggregation in the brain. Aβ can aggregate into oligomers, pre-fibrils and mature fibers. Aβ oligomers are the most neurotoxic aggregates, and the most reported forms are Aβ dimers, trimers, ADDLs, etc. Aβ oligomers can be divided into two types according to their molecular weight, low molecular weight and high molecular weight, and high molecular weight oligomers have greater neurotoxicity. Aβ dimers are the smallest Aβ oligomers, and it is generally believed that Aβ dimers are the basic building blocks of Aβ oligomers. Aβ dimers show an elevated level in the brains of AD patients and AD transgenic mice, and are stable in SDS and strong denaturants, with certain neurotoxicity. In addition to Aβ dimers, Aβ trimers are also considered to be the aggregation units of various Aβ oligomers such as hexamers and dodecamers. Aβ trimers appear early in the brains of AD patients and AD transgenic mice, but there is no significant correlation between Aβ trimers and Aβ plaque deposition, and their toxicity is still controversial. In addition, Aβ oligomers can also form spherical oligomers (ASPD) with a diameter of about 12 nm and diffusible oligomers (ADDL) with a diameter of 5-6 nm after further aggregation. Both of these polymer forms are considered to be Aβ oligomers with unique conformation and neurotoxicity. At present, although a variety of Aβ oligomer forms have been discovered, it is still unknown which Aβ oligomers are the most toxic ones and play a major role in the occurrence and development of AD. This may be the reason why the development of drug targeting Aβ oligomers has not been successful. In addition, the instability of Aβ oligomers limits the extraction and research of a certain single configuration of oligomers. Therefore, improving the extraction and detection technology of Aβ oligomers is one of the keys to clarify the types of Aβ oligomers and their corresponding functions. Moreover, the methods for specifically detecting oligomers, especially toxic oligomers, are very limited, and it is particularly difficult to obtain antibodies that specifically bind to toxic amyloid oligomers, which seriously limits the clinical detection and development of therapeutic agents for Aβ oligomers.

A large number of studies have shown that the severity of neurodegenerative diseases is closely related to the level of amyloid oligomers in the patient's brain. Aβ oligomers play a key role in the occurrence and development of AD by causing functional neuronal death, cognitive impairment and dementia. However, there are many types of Aβ oligomers, which can affect the function of central nervous system through various mechanisms. The toxicity of various Aβ oligomers differ from each other, and there are also great differences in Aβ oligomer's size, conformation, aggregation mode, toxicity and timing of appearance in the brain. Although many studies have shown that some Aβ oligomers, such as dimers, trimers, ADDL, etc., can play a neurotoxic role, our current understanding on the Aβ oligomer form that really plays a key pathogenic role is not deep enough. Therefore, the study of Aβ oligomer toxicity should be accurate to the type of Aβ oligomers and the corresponding toxic mechanism, rather than oligomer mixtures. Determining the key toxic oligomers that are closely related to the occurrence and development of AD can provide ideal markers for early warning and early diagnosis of AD, and can also provide ideal precise targets for the treatment of AD.

Immunotherapy has always been the focus of research in the field of AD treatment. Aβ antibodies and vaccines have gone through the development of three generations of products targeting the overall molecule of Aβ, the N-terminus of Aβ, and the spatial structure of Aβ aggregates. In recent years, dozens of antibodies and vaccines targeting Aβ have entered clinical trials. Although these preparations have shown good therapeutic effects in the animal testing phase, significantly improving the cognitive level of AD transgenic animals and reducing the amount of senile plaques and other pathological changes in the animals' brains, however in AD clinical trials, due to poor therapeutic effects or serious side effects, few have passed clinical trial phase III so far. In June 2021, the U.S. Food and Drug Administration (FDA) approved Aduhelm (aducanumab) through the accelerated approval program. This antibody can specifically recognize Aβ oligomers and has shown certain efficacy in clinical trials. However, the antibody still has adverse reactions such as brain edema and inflammation, and the efficacy of the antibody is also controversial.

Therefore, there is still a need in the field to identify the specific Aβ oligomer that plays a key role in the occurrence and development of AD and apply it to treat and/or prevent AD.

### Summary of the present invention

The first aspect of the present invention relates to an isolated Aβo*3F, wherein the Aβo*3F is an Aβ oligomer specifically bound by the antibody 3F or variant thereof, wherein total molecular weight of the Aβo*3F is about 588 kDa based on size exclusion chromatography (SEC) analysis, the light and heavy chain CDR sequences of the antibody 3F are as set forth in SEQ ID NOs: 17-22, respectively, and the variant is K98R mutant or the antibody 7B, of which the light and heavy chain CDR sequences are as set forth in SEQ ID NOs: 25-30 or SEQ ID NOs: 32-35 and KAS, respectively.

In some embodiments, the isolated Aβo*3F is prepared *in vitro.*

The second aspect of the present invention relates to a composition comprising the Aβo*3F as described above.

In some embodiments, the composition is an immunogenic composition, a pharmaceutical composition, or a vaccine.

In some embodiments, the composition further comprises a pharmaceutically acceptable excipient and/or adjuvant.

In some embodiments, the composition further comprises additional active ingredients, preferably, the additional active ingredients are other forms of Aβ oligomers, pre-fibrils and mature fibers, such as Aβ dimers, trimers, hexamers, dodecamers, ASPD, ADDL, etc. Preferably, the additional active ingredients are binding agents that specifically bind to the Aβo*3F, preferably, the binding agents that specifically bind to the Aβo*3F are the antibodies 3F, K98R and 7B, more preferably, the binding agents that specifically bind to the Aβo*3F are in a separated state with the Aβo*3F for simultaneous or sequential administration. In some embodiments, the composition further comprises additional drugs for the treatment of mild cognitive impairment (MCI) and AD.

The third aspect of the present invention relates to an *in vitro* method for preparing the Aβo*3F as described above, comprising the following steps: dissolving Aβ42, Aβ40 or other forms of Aβ in 50 mM NaOH at a concentration of 1 mg/mL, vortexing for 3-5 min, sonicating for 1 min, and then diluting to 10 µM with pre-cooled PBS; centrifuging at 21,000 g for 30-40 min at 4°C, discarding the precipitate (approximately 5% of the starting volume ) to obtain Aβ monomers; incubating the Aβ monomers statically at 25°C for 2 days, and incubating them with Protein A magnetic beads cross-linked with the antibody 3F at 4°C overnight; on the next day, the magnetic beads were washed three times with 0.1% PBST, eluted with 20-100 mM glycine (pH 2.0) for 3-5 min, eluted twice, and the eluate was neutralized to pH 7 with 1 M Tris to obtain the Aβo*3F.

The fourth aspect of the present invention relates to use of Aβo*3F as a target in the preparation of a medicament for preventing and/or treating MCI and/or AD in a subject, wherein the Aβo*3F is an Aβ oligomer specifically bound by the antibody 3F or variant thereof, wherein total molecular weight of the Aβo*3F is about 588 kDa based on size exclusion chromatography (SEC) analysis, the light and heavy chain CDR sequences of the antibody 3F are as set forth in SEQ ID NOs: 17-22, respectively, and the variant is K98R mutant or the antibody 7B, of which the light and heavy chain CDR sequences are as set forth in SEQ ID NOs: 25-30 or SEQ ID NOs: 32-35 and KAS, respectively.

In some embodiments, the drug is an antiserum, an antibody, or a small molecule drug that specifically binds to Aβo*3F. Preferably, the antibody is a polyclonal antibody or a monoclonal antibody or an antigen-binding fragment thereof.

Another aspect of the present invention relates to a binding agent that specifically binds to the Aβo*3F as described above, wherein the binding agent is the antibodies 3F, K98R and 7B.

In some embodiments, the binding agent is an antiserum, an antibody, or a small molecule drug that specifically binds to the Aβo*3F. Preferably, the antibody is a polyclonal antibody or a monoclonal antibody or an antigen-binding fragment thereof. In some embodiments, the antibody is a single-chain antibody. In some embodiments, the heavy chain CDR sequences of the antibody are as set forth in SEQ ID NOs: 25-27, and the light chain CDR sequences are as set forth in SEQ ID NOs: 28-30, respectively.

In another aspect, the present invention relates to a method for preparing the binding agent as described above, comprising the steps of immunizing an animal with an immunologically effective amount of the Aβo*3F as described above, or using the Aβo*3F as described above as a substrate to screen phage display peptide libraries, antibody libraries, compound libraries, etc.

In some embodiments, the animal is a mouse, a rat, a guinea pig, a rabbit, a sheep, a goat, a monkey, a horse, a cow, an alpaca, or a non-human primate.

Another aspect of the present invention relates to a composition comprising the binding agent as described above or the binding agent obtained by the method as described above, preferably, the composition further comprises the Aβo*3F as described above, more preferably, the binding agent that specifically binds to the Aβo*3F and the Aβo*3F are in a separated state for simultaneous or sequential administration. In some embodiments, the composition further comprises additional neurodegenerative disease therapeutic agent, preferably, the additional neurodegenerative disease therapeutic agent is selected from the group consisting of acetylcholinesterase inhibitors such as donepezil, galantamine, carbastine, etc., and aspartate receptor antagonists such as memantine, etc.

In another aspect, the present invention relates to use of the Aβo*3F, the composition or the binding agent as described above in the manufacture of a medicament for preventing and/or treating MCI and/or AD in a subject.

In yet another aspect, the present invention relates to the Aβo*3F, the composition or the binding agent as described above for use as a medicament for preventing and/or treating MCI and/or AD in a subject.

Still another aspect of the present invention relates to a method for preventing and/or treating MCI and/or AD in a subject, comprising administering to the subject a preventively and/or therapeutically effective amount of the Aβo*3F, the composition or the binding agent as described above.

Another aspect of the present invention relates to use of Aβo*3F as a diagnostic target for MCI and/or AD in a subject, wherein the Aβo*3F is an Aβ oligomer in a subject specifically bound by the antibody 3F or a variant thereof, wherein total molecular weight of the Aβo*3F is approximately 588 kDa based on size exclusion chromatography (SEC) analysis, and the light and heavy chain CDR sequences of the antibody 3F are as set forth in SEQ ID NOs: 17-22, respectively, and the variant is K98R mutant or the antibody 7B, of which the light and heavy chain CDR sequences are as set forth in SEQ ID NOs: 25-30 or SEQ ID NOs: 32-35 and KAS, respectively. In other words, the Aβo*3F in the subject as described above can be used as a biomarker for diagnosing the presence or absence of MCI and/or AD in the subject. A person skilled in the art would know how to diagnose whether a subject suffers from MCI and/or AD and/or is at risk of suffering from MCI and/or AD based on Aβo*3F as a biomarker. In some embodiments, the diagnosis can be performed using an antibody that specifically binds to the Aβo*3F, such as the antibodies 3F, K98R, or 7B.

Another aspect of the present invention relates to use of Aβo*3F as a target for the prevention and/or treatment of MCI and/or AD in a subject, wherein the Aβo*3FAβo*3F is an Aβ oligomer in a subject specifically bound by the antibody 3F or a variant thereof, wherein total molecular weight of the Aβo*3F is approximately 588 kDa based on size exclusion chromatography (SEC) analysis, and the light and heavy chain CDR sequences of the antibody 3F are as set forth in SEQ ID NOs: 17-22, respectively, and the variant is K98R mutant or the antibody 7B, of which the light and heavy chain CDR sequences are as set forth in SEQ ID NOs: 25-30 or SEQ ID NOs: 32-35 and KAS, respectively. In other words, the Aβo*3F in the subject as described above can be used as a target for the prevention and/or treatment of MCI and/or AD in the subject. A person skilled in the art would know how to prevent and/or treat MCI and/or AD in a subject based on the Aβo*3F as a target. In some embodiments, the prevention and/or treatment can be performed using antibodies that specifically bind to the Aβo*3F, such as the antibodies 3F, K98R, or 7B, or small molecule drugs.

In some embodiments, the subject is a human, a non-human primate, a cat, or a dog.

In other words, in the preliminary research, the present inventors used phage display technology to firstly screen out the fully human single-chain antibody W20 (see CN101463082A), and the antibody 3F (having the amino acid sequence as set forth in SEQ ID No. 23) is an improved form of the antibody 20. Compared with the antibody 20, the antibody 3F's affinity to Aβ oligomers is significantly improved, and the antibody 3F can more significantly inhibit the aggregation of Aβ and the neuronal cytotoxicity induced by Aβ oligomers, more effectively improve the cognitive and memory functions of AD model mice, and reduce pathological changes in the mouse brain. More significantly, the Aβ oligomers specifically recognized by this antibody are super toxic oligomers, they are the main toxic component in the Aβ oligomer mixture, have a strong pathogenic effect, and play an important role in the occurrence and development of AD. The highly toxic oligomer recognized by the antibody 3F is present in the CSF, blood and/or brain tissue of AD patients and AD-derived MCI patients, and its level shows extremely significant differences in the CSF, blood and/or brain of the three human groups, i.e., AD patients, MCI patients and healthy elderly patients, so that AD patients, MCI patients and healthy elderly people can be accurately distinguished. This toxic oligomer also exists in AD transgenic mice and is directly related to the pathogenesis of AD transgenic mice. In the present invention, the highly toxic Aβ oligomer recognized by the antibody 3F is called AβO*3F. It can be separated from the Aβ oligomer mixture by immunoprecipitation (with the antibody 3F). Its typical characteristics are Aβ high molecular weight oligomers, based on size exclusion chromatography (SEC) analysis, its molecular weight is approximately 588 kDa and its diameter is approximately 10 nm. It has a strong toxic effect on neurons, and its toxicity is more than 200 times more toxic than the Aβ oligomer mixture. The Aβo*3F can activate microglia and/or astrocytes and secrete a large number of inflammatory factors. More importantly, the present invention successfully prepared the Aβo*3F *in vitro.* The Aβo*3F prepared *in vitro* is consistent in composition with the product immunoprecipitated from the cerebrospinal fluid or plasma of AD patients using the antibody 3F, and they have the same physical and chemical properties and functions. The Aβo*3F prepared *in vitro* can be used, for example, as an immunogen to immunize target organisms, to obtain an antiserum, polyclonal antibodies or monoclonal antibodies against the Aβo*3F, and to treat and/or prevent AD patients or MCI patients. It can also be used, for example, as a therapeutic or prophylactic vaccine to immunize a target patient to treat and/or prevent AD or MCI in the target patient. The Aβo*3F or composition thereof described herein provides new therapeutic and/or preventive targets and new therapeutic and/or preventive approaches for AD or MCI.

### BRIEF DESCRIPTION OF THE DRAWINGS

Figure 1 shows a schematic diagram of immunoprecipitation preparation of the Aβo*3F, Aβ*6E10 and Aβ-ID.
Figure 2 shows the molecular weight and morphology of the Aβo*3F:
   A: the Aβ aggregation state detected by ThT;
   B: the binding of oligomers obtained under different incubation conditions with the antibodies 3F, A11 and 6E10 detected by Dot blot;
   C: the affinity of Aβos obtained from 0-4 days of 10 µM Aβ incubation with the antibody 3F;
   D: the sAβo*3F band distribution detected by Western blot;
   E: Size exclusion chromatography (SEC) analysis of the molecular weight of sAβo*3F; SEC analysis of sAβo*3F and SEC standards (Superdex 200 10/300), SEC standards: 1. Thyroglobulin (669 kDa), 2. Ferritin (440 kDa), 3. Aldolase (158 kDa), 4. Conalbumin (75 kDa), 5. Ovalbumin (44 kDa), 6. Carbonic anhydrase (29 kDa);
   F: Fitting linear equation (Y) and correlation coefficient (r2) according to the molecular weight and elution volume of SEC standards;
   G: Western blot detection of mAβo*3F band distribution;
   H: SEC analysis of mAβo*3F molecular weight;
   I: Western blot detection of hAβo*3F band distribution.

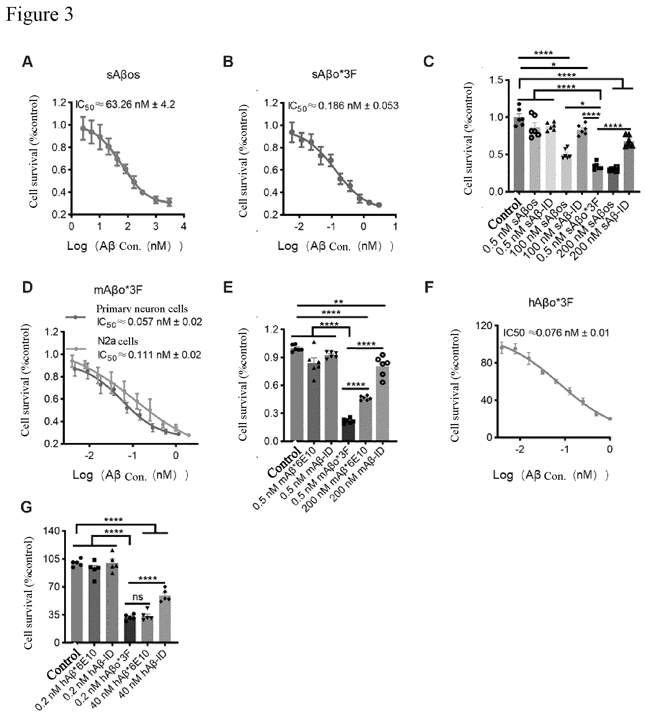

Figure 3 shows the neurotoxic effect of the Aβo*3F:
   A: IC50 of sAβos cytotoxicity to N2a cells;
   B: IC50 of sAβo*3F cytotoxicity to N2a cells;
   C: MTT method was used to compare the neurotoxicity of sAβos, sAβo*3F and sAβ-ID to N2a cells;
   D: IC50 of mAβo*3F toxicity to N2a cells and primary neurons;
   E: MTT method was used to compare the cytotoxicity of mAβ*6E10, mAβo*3F and mAβ-ID on primary neurons;
   F: IC50 of hAβo*3F toxicity to primary neurons;
   G: MTT method was used to compare the cytotoxicity of hAβ*6E10, hAβo*3F and hAβ-ID to primary neurons.

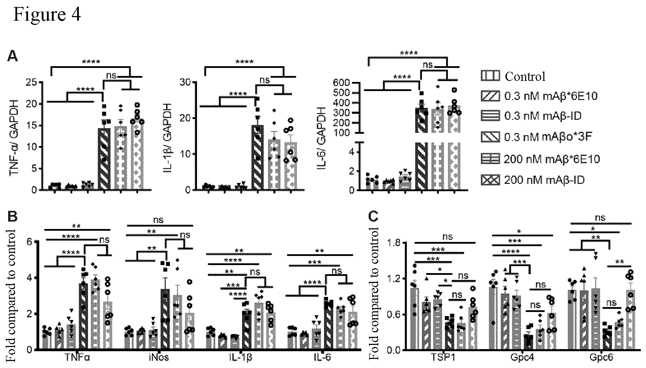

Figure 4 shows the effect of Aβo*3F on the cytokine expression level of glial cells:
   A: Effects of mAβo*3F on TNF-α, IL-1β, and IL-6 expression levels in microglia;
   B: Effects of mAβo*3F on TNF-α, iNos, IL-1β, and IL-6 expression levels in primary astrocytes;
   C: Effect of mAβo*3F on TSP1, Gpc4 and Gpc6 expression levels in primary astrocytes.

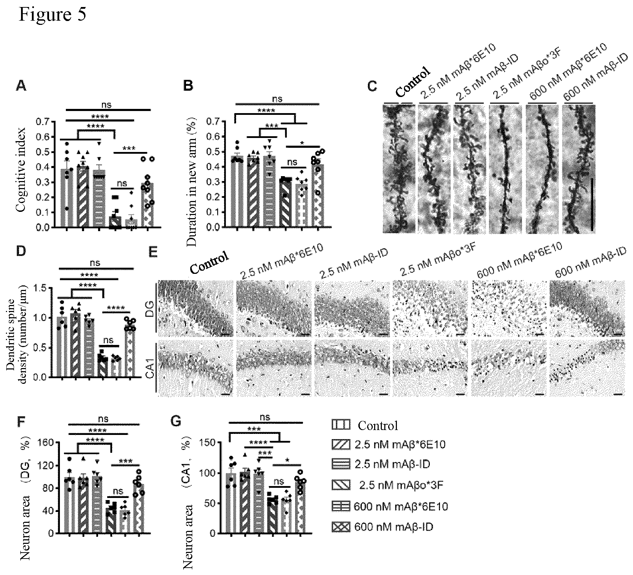

Figure 5 shows the effects of the Aβo*3F on mouse cognition function and its damaging effects on brain neurons:
   A: Cognitive index of mice in each group in the novel object recognition test;
   B: The duration of each group of mice in the new arm in the Y-maze test;
   C: Golgi staining to detect the density of dendritic spines in mouse neurons;
   D: Statistical analysis of dendritic spine density in C;
   E: Nissl staining to detect the number of neurons in mouse hippocampus, scale bar: 20 µm;
   F, G: The number of neurons in the DG area (F) and CA1 area (G) was statistically analyzed using Image J software.

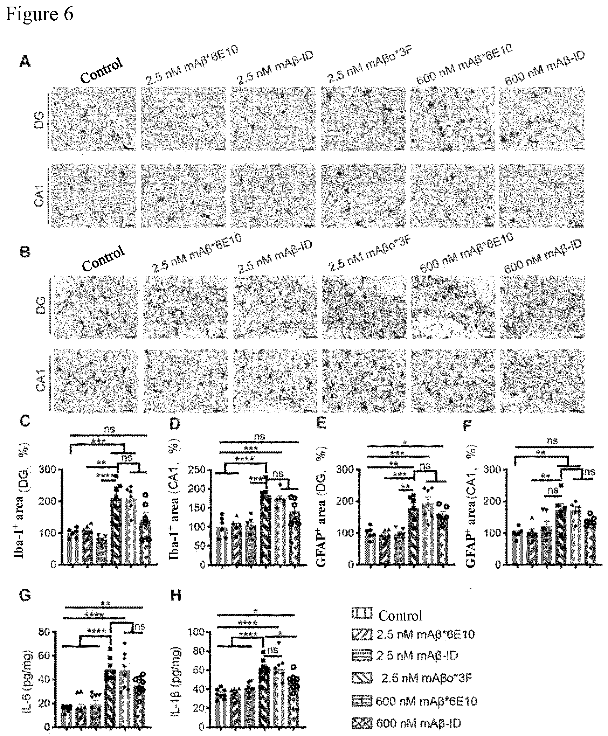

Figure 6 shows that the Aβo*3F activates glial cells in the mouse brain to produce neuroinflammation:
   A: Immunohistochemistry assay to detect the activation of microglia in the DG and CA1 regions of the mouse hippocampus, scale bar: 20 µm;
   B: Immunohistochemistry assay to detect the activation of astrocytes in the DG and CA1 regions of the mouse hippocampus, scale bar: 20 µm;
   C, D: The area of Iba-1-positive microglia in the DG region (C) and CA1 region (D) was quantified using Image J software;
   E, F: The area of GFAP-positive astrocytes in the DG region (E) and CA1 region (F) was quantified using Image J software;
   G, H: The expression levels of IL-6 (G) and IL-1β (H) in the mouse hippocampus were detected by ELISA.

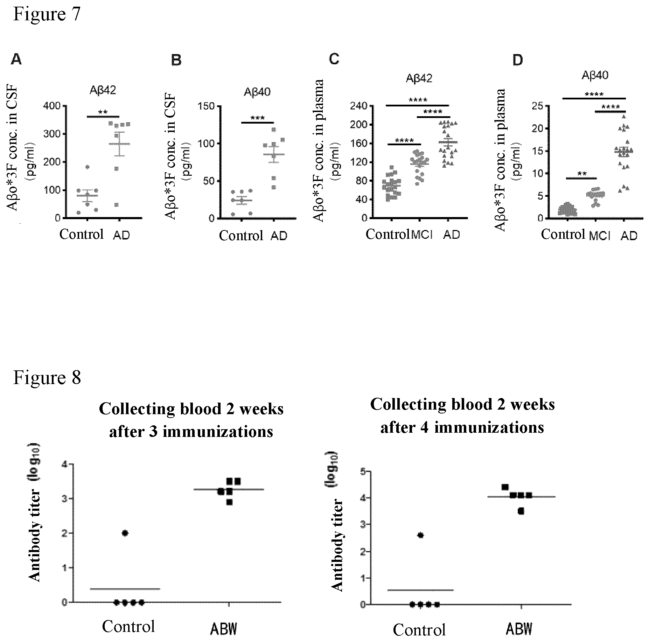

Figure 7 shows the electrochemiluminescence method for detecting the Aβo*3F levels in CSF and plasma of AD patients:
   A, B: the corresponding levels of Aβ42 (A) and Aβ40 (B) in the Aβo*3F isolated from CSF of AD patients and healthy population (Con) were detected by MSD method;
   C, D: the corresponding levels of Aβ42 (C) and Aβ40 (D) in the Aβo*3F isolated from the plasma of patients with mild cognitive impairment (MCI), AD and healthy population (Con) were detected by MSD method.
Figure 8 shows that antibodies induced by ABW vaccine specifically bind to Aβ oligomers. Blood was collected two weeks after the third and fourth immunization of the animals, and the antibody titer in the serum was measured by ELISA.

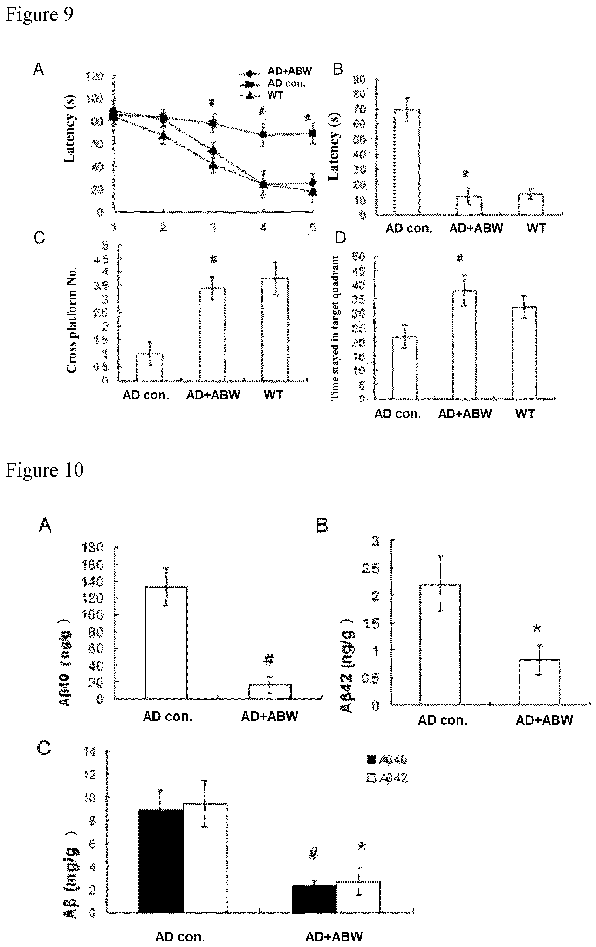

Figure 9 shows that ABW vaccine significantly improved the memory of AD transgenic mice in the water maze test. (A) During the training phase, the latency of the mouse to find the platform; (B-D) After the platform was removed, the latency of the mouse to reach the platform (B), the number of times the mouse crossed the platform location (C) and the time it stayed in the target quadrant (D).
Figure 10 shows that ABW vaccine reduces the level of Aβ in the brain of AD mice. The levels of soluble Aβ40 (A), soluble Aβ42 (B), and insoluble Aβ40 and Aβ42 (C) in the brain of AD mice treated with ABW vaccine were measured by ELISA.

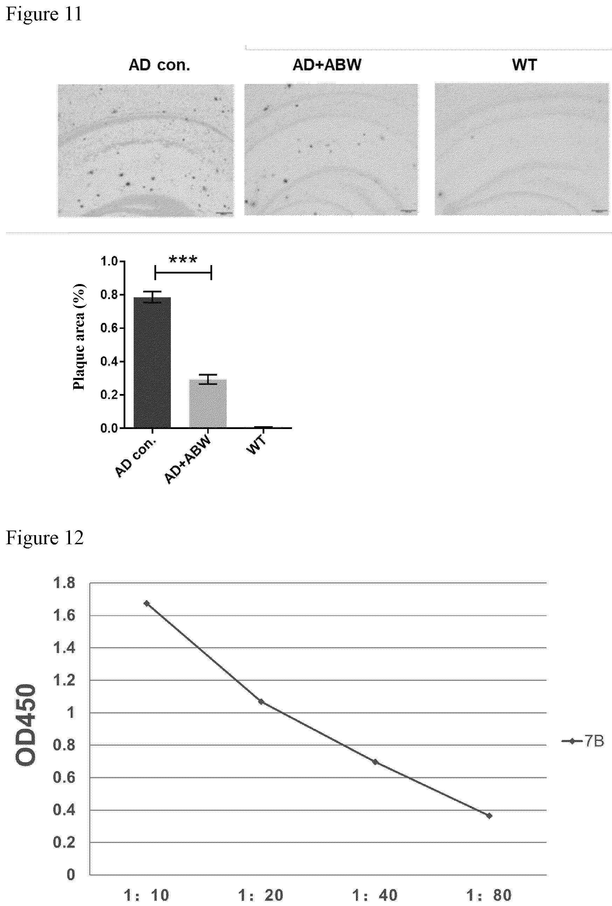

Figure 11 shows that ABW vaccine reduces Aβ plaques in the brain of AD mice. Immunohistochemical staining with 6E10 antibody, scale bar = 100 µm; quantitative analysis of Aβ plaque staining area.
Figure 12 shows the ELISA results of the antibody 7B binding to the Aβo*3F.

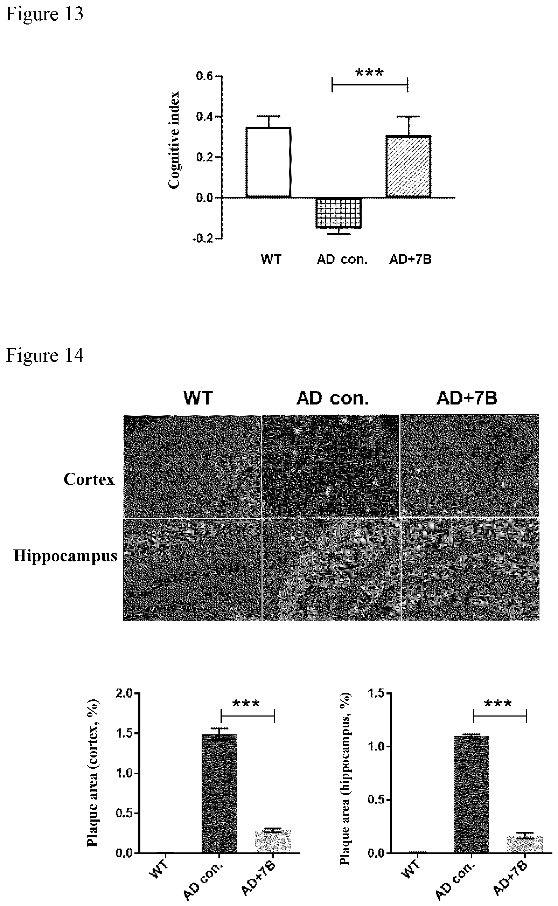

Figure 13 shows that the antibody 7B significantly improved the cognitive ability of AD transgenic mice. Cognitive index of mice in each group in the novel object recognition test.
Figure 14 Thioflavin S (THS) staining showed that the antibody 7B reduced Aβ plaques in the brain of AD mice.

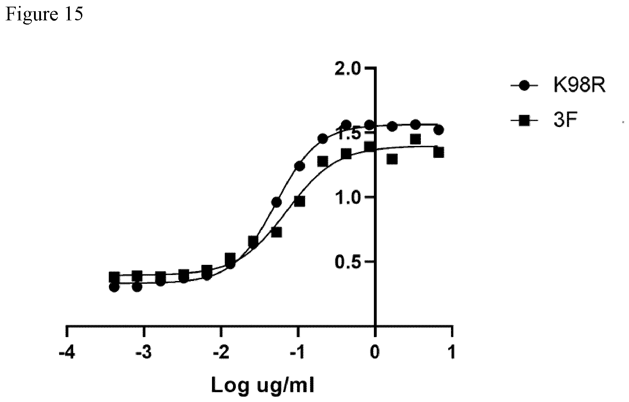

Figure 15 shows that after the K at position 98 of the antibody 3F heavy chain mutated to R, the affinity for binding to the Aβo*3F was further improved, suggesting that the combination of this single-chain antibody and Aβ oligomer may have better activity.

### DETAILED DESCRIPTION

### Definitions

Unless otherwise indicated, the terms used in the claims and specification are defined as shown below.

Unless otherwise defined herein, the scientific and technical terms used in conjunction with the methods and compositions of the present invention as described herein shall have the meanings commonly understood by those of ordinary skill in the art. In addition, unless the context otherwise requires, singular terms shall include plural terms, and plural terms shall include singular terms. Typically, the nomenclature and techniques used in conjunction with the following fields are those well known and commonly used in the art: biochemistry, immunology enzymology, molecular and cell biology, microbiology, genetics, and polypeptide chemistry, as described herein.

The methods and techniques described herein are generally performed according to conventional methods well known in the art and as described in various general and more specific references that are cited and discussed throughout the present specification, unless otherwise indicated. See, e.g., Sambrook et al., Molecular Cloning: A Laboratory Manual, 2nd ed., Cold Spring Harbor Laboratory Press, Cold Spring Harbor, NY (1989); Ausubel et al., Current Protocols in Molecular Biology, Greene Publishing Associates (1992, and supplements through 2002); Harlow and Lane, Antibodies: A Laboratory Manual, Cold Spring Harbor Laboratory Press, Cold Spring Harbor, NY (1990); Taylor and Drickamer, Introduction to Glycobiology, Oxford Univ. Press (2003); Worthington Enzyme Manual, Worthington Biochemical Corp., Freehold, NJ; Handbook of Biochemistry: Section A Proteins, Volume I, CRC Press (1976); Handbook of Biochemistry: Section A Proteins, Volume II, CRC Press (1976); Essentials of Glycobiology, Cold Spring Harbor Laboratory Press (1999).

All the publications, patents, and other references mentioned herein are expressly incorporated by reference in their entirety.

Unless otherwise indicated, the following terms shall be understood to have the following meanings.

When used in conjunction with a number, the term "about" as used herein refers to any number that is within ±1, ±5, or ±10% of the referenced number.

The term "Aβo*3F" as used herein refers to an Aβ oligomer specifically bound by the antibody 3F, and based on size exclusion chromatography (SEC) analysis, its molecular weight is about 588 kDa, and the light and heavy chain CDR sequences of the antibody 3F are as set forth in SEQ ID NOs: 17-22, respectively. In some embodiments, the size exclusion chromatography (SEC) analysis is performed using a Superdex 200 10/300 GL molecular exclusion chromatography column.

In some embodiments, the Aβo*3F is an isolated Aβo*3F. In some embodiments, the Aβo*3F is prepared *in vitro.* In some embodiments, the Aβo*3F is prepared *in vitro* and purified. In some embodiments, the purity of the purified Aβo*3F is at least 1% by weight of the purified product, such as 1.5-99.9%, preferably 15-99.9%, 25-99.9%, 50-99.9%, 65-99.9%, 95-99.9%, 95-99.9%, such as at least 2, 3, 4, 5, 6, 7, 8, 9, 10, 12, 14, 16, 18, 20, 25, 30, 35, 40, 45, 50, 55, 60, 65, 70, 75, 80, 85, 90, 91, 92, 92, 93, 94, 95, 96, 97, 98, 99, 99.5% or more. In some embodiments, the purification is affinity purification, for example, using a monoclonal antibody specific for the Aβo*3F, such as the antibody 3F. In some embodiments, the *in vitro* preparation of the Aβo*3F is performed as follows:

Aβ42, Aβ40 or other forms of Aβ were dissolved in 50 mM NaOH at a concentration of 1 mg/mL, vortexed for 3-5 min, sonicated for 1 min, and then diluted to 10 µM with pre-cooled PBS. The dilutes were centrifuged at 21,000 g for 30-40 min at 4°C, the precipitate (about 5% of the starting volume) was discarded, and Aβ monomers were obtained. Aβ monomers were statically incubated at 25°C for 2 days, and incubated with Protein A magnetic beads cross-linked with the antibody 3F at 4°C overnight. The next day, the magnetic beads were washed three times with 0.1% PBST, eluted with 20-100 mM glycine (pH 2.0) for 3-5 min, eluted twice, and the eluate was neutralized to pH7 with 1 M Tris to obtain the Aβo*3F.

In some embodiments, provided herein are a composition comprising the Aβo*3F, wherein the weight of the Aβo*3F is 0.01-99.9%, for example, 0.05, 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 6.0, 7.0, 8.0, 9.0, 10, 15, 20, 25, 30, 35, 40, 45, 50, 60, 70, 80, 90, 91, 92, 93, 94, 95, 96, 97, 98, 99% or more. In some embodiments, the composition provided herein comprises the Aβo*3F, wherein the amount of the Aβo*3F present in the composition is typically in the range of 1-200 µg/mL, suitably 1-100 µg/mL, suitably 5-50 µg/mL, preferably 8 to 40 µg/mL, more preferably 16-32 µg/mL, for example 16, 18, 20, 22, 24, 26, 28, 30 or 32 µg/mL. The dose by volume suitable for human use is typically 0.25 to 1.5 ml. In one embodiment, the human dose is 0.5 mL. In a further embodiment, the human dose is higher than 0.5 ml, such as 0.6, 0.7, 0.8, 0.9 or 1 mL. In a further embodiment, the human dose is 1 ml - 1.5 ml.

In some embodiments, the composition is an immunogenic composition. As used herein, "immunogenic composition" refers to a composition that can induce an immune response in a host or subject to which the composition is applied. The immunogenic composition comprises an immunologically effective amount of the AβO*3F of the present invention and any other components. "Immunologically effective amount" means that the amount is administered to an individual as a single dose or as part of a series for treatment or prevention, or a detectable amount of polyclonal antibodies or polyclonal antibodies can be produced. The amount varies depending on the health and physical condition of the individual to be treated, age, desired degree of protection, vaccine formulation, and other relevant factors. The amount will fall into a relatively wide range that can be determined by routine experiments.

In some embodiments, the immunogenic composition may further comprise a pharmaceutically acceptable carrier. In some embodiments, the composition is a pharmaceutical composition further comprising a pharmaceutically acceptable carrier. The immunogenic composition of the present invention may be formulated into a pharmaceutical composition before being administered to a subject. The present invention also provides a vaccine comprising the immunogenic composition of the present invention and a pharmaceutically acceptable excipient or carrier. As used herein, "pharmaceutically acceptable carrier" refers to a diluent, adjuvant, excipient or vehicle that is administered with the composition and is non-toxic and should not interfere with the efficacy of the active ingredient. Vaccine formulations are generally described in Vaccine Design ("The subunit and adjuvant approach" (ed. Powell MF & Newman MJ) (1995) Plenum Press New York).

The immunogenic composition or vaccine of the present invention may be included in a container, package or dispenser together with instructions for administration. The present invention provides a kit comprising: (i) a first container comprising an immunogenic composition or vaccine of the present invention; and optionally, (ii) a second container comprising an adjuvant as described herein.

Also contemplated herein are compositions that optionally further comprise other forms of Aβ oligomers, pre-fibrils and mature fibrils, such as Aβ dimers, trimers, hexamers, dodecamers, ASPD, ADDL, and the like.

In certain embodiments, the composition described herein (e.g., a pharmaceutical composition and/or an immunogenic composition) comprises an adjuvant or is administered in combination with an adjuvant. The adjuvant for administration in combination with the composition described herein can be administered prior to (e.g., within 72 hours, 48 hours, 24 hours, 12 hours, 6 hours, 2 hours, 1 hour, 10 minutes), simultaneously with, or after (e.g., within 72 hours, 48 hours, 24 hours, 12 hours, 6 hours, 2 hours, 1 hour, 10 minutes) administration of the composition.

In certain embodiments, the composition described herein does not include an adjuvant and is not administered in combination with an adjuvant.

The immunogenic composition disclosed herein typically contains one or more pharmaceutically acceptable carriers and/or excipients. Pharmaceutically acceptable carriers and excipients are well known in the art and can be selected by those skilled in the art. The adjective "pharmaceutically acceptable" indicates that the object mentioned is suitable for administration to a subject (e.g., a human or animal subject). Remington's Pharmaceutical Sciences, E. W. Martin, Mack Publishing Co., Easton, Pa., The 15th edition (1975) describes compositions and formulations (including diluents) suitable for the pharmaceutical delivery of therapeutic and/or prophylactic compositions, including immunogenic compositions.

For example, the carrier or excipient may advantageously include a buffer. Optionally, the carrier or excipient also contains at least one component that stabilizes solubility and/or stability. Examples of solubilizers/stabilizers include detergents, such as lauric sarcosine and/or Tween. Many pharmaceutically acceptable carriers and/or pharmaceutically acceptable excipients are known in the art and are described, for example, in Remington's Pharmaceutical Sciences, E. W. Martin, Mack Publishing Co., Easton, Pa., 5th Edition (1975). Thus, one skilled in the art can select appropriate excipients and carriers to produce a formulation suitable for delivery to a subject via a chosen route of administration.

Suitable excipients include, but are not limited to: glycerol, polyethylene glycol (PEG), sorbitol, trehalose, sodium N-lauroylsarcosine, L-proline, non-detergent sulfobetaine, guanidine hydrochloride, urea, trimethylamine oxide, KCl, Ca²⁺, Mg²⁺, Mn²⁺, Zn²⁺ and other divalent cation-related salts, dithiothreitol, dithioerythritol and 13-mercaptoethanol. Other excipients can be a detergent (including: Tween80, Tween20, Triton X-00, NP-40, Empigen BB, Octyl Glucoside, Lauroyl Maltoside, Zwittergent 3-08, Zwittergent 3-0, Zwittergent 3-2, Zwittergent 3-4, Zwittergent 3-6, CHAPS, sodium deoxycholate, sodium dodecyl sulfate, hexadecyltrimethylammonium bromide).

Optionally, the immunogenic composition of the present invention can be formulated to contain other components, including, for example, adjuvants, stabilizers, pH modifiers, preservatives, and the like.

As used herein, the term "adjuvant" refers to a composition that enhances the immune response to an immunogen. Composition according to the invention comprising an adjuvant can be used as vaccines, for example, for human subjects. Adjuvants accelerate, prolong and/or enhance the quality and/or intensity of the immune response to an antigen/immunogen compared to administration of the antigen alone, thus reducing the amount of antigen/immunogen necessary in any given vaccine, and/or reducing the frequency of injection necessary to generate an adequate immune response to the target antigen/immunogen.

Examples of adjuvants that can be used in the context of the composition of the present invention include inorganic adjuvants (e.g. inorganic metal salts such as aluminum phosphate or aluminum hydroxide), gel-like precipitates of aluminum hydroxide (alum); AlPO₄; hydrogels; bacterial products from the outer membrane of Gram-negative bacteria, in particular monophosphoryl lipid A (MPLA), lipopolysaccharide (LPS), muramyl dipeptide and its derivatives; Freund's incomplete adjuvant; liposomes, in particular neutral liposomes, liposomes containing the composition and optionally cytokines; AS01B, AS01E, AS02; nonionic block copolymers; ISCOMATRIX adjuvant; unmethylated DNA containing CpG dinucleotides (CpG motifs), in particular CpG ODN having a phosphorothioate (PTO) backbone, or CpG ODN (CpG PO ODN) having a phosphodiester (PO) backbone; synthetic lipopeptide derivatives, especially Pam3Cys; lipoarabinomannan; peptidoglycan; zymosan; heat shock protein (HSP), especially HSP 70; dsRNA and its synthetic derivatives, especially poly I: poly C; polycationic peptides, especially poly-L-arginine; paclitaxel; fibronectin; flagellin; imidazoquinoline; cytokines with adjuvant activity, especially GM-CSF, interleukins (IL-2, IL-6, IL-7, IL-18), type I and II interferons, especially interferon-γ, TNF-α; 2,5-dihydroxyvitamin D3 (calcitriol); and synthetic oligopeptides, especially MHCII-presenting peptides. Nonionic block polymers containing polyoxyethylene (POE) and polyoxypropylene (POP), such as POE-POP-POE block copolymers, can be used as adjuvants.

Additional examples of adjuvants include inorganic adjuvants (e.g., inorganic metal salts such as aluminum phosphate or aluminum hydroxide), organic adjuvants (e.g., saponins such as QS21 or squalene), oil-based adjuvants (e.g., Freund's complete adjuvant and Freund's incomplete adjuvant), cytokines (e.g., IL-1β, IL-2, IL-7, IL-12, IL-18, GM-CFS, and INF-γ), particulate adjuvants (e.g., immunostimulatory complexes (ISCOMS), liposomes, biodegradable microspheres, virosomes, bacterial adjuvants (e.g., monophosphoryl lipid A, such as 3-de-O-acylated monophosphoryl lipid A (3D-MPL) or muramyl peptides), synthetic adjuvants (e.g., monophosphoryl lipid A (MPL), particularly 3-de-O-acylated monophosphoryl lipid A (3D-MPL), and muramyl peptide analogs or synthetic lipid A, and synthetic polynucleotide adjuvants, such as polyarginine or polylysine.

Saponins are also suitable adjuvants, for example, saponin Quil A (derived from the bark of the South American soap tree Molina) and its fractions. Purified fractions of Quil-A are also known as immunostimulants, such as squalene, QS21, QS 17 and QS7 (non-hemolytic fractions of Quil-A). Combinations of QS21 and polysorbates or cyclodextrins are also suitable.

Another example of an adjuvant is an immunostimulatory oligonucleotide containing an unmethylated cytosine-guanosine dinucleotide motif ("CpG") present in DNA. When administered by systemic and mucosal routes, CpG is referred to as an adjuvant. When formulated in a vaccine, it can be administered in free solution with the free antigen, or covalently conjugated to the antigen, or formulated with a carrier such as aluminum hydroxide.

Activation of specific receptors can stimulate an immune response. Such receptors are known to the skilled person and include, for example, cytokine receptors, particularly type I cytokine receptors, type II cytokine receptors, TNF receptors; and vitamin D receptors acting as transcription factors; and Toll-like receptor 1 (TLR1), TLR2, TLR3, TLR4, TLR5, TLR-6, TLR7 and TLR9. Agonists of such receptors have adjuvant activity, i.e., are immunostimulatory. Other suitable adjuvants include alkyl aminoglucosyl phosphates (AGP) or pharmaceutically acceptable salts of AGP. Some AGPs are TLR4 agonists, and some are TLR4 antagonists. The adjuvant of the composition of the present invention may be one or more Toll-like receptor agonists. In a more preferred embodiment, the adjuvant is a Toll-like receptor 4 agonist. In a particularly preferred embodiment, the adjuvant is a Toll-like receptor 9 agonist.

Adjuvants such as those described above can be formulated with carriers such as liposomes, oil-in-water emulsions, and/or metallic salts (including aluminum salts, such as aluminum hydroxide). For example, 3D-MPL can be formulated with aluminum hydroxide or oil-in-water emulsions; QS21 can be formulated with cholesterol-containing liposomes, oil-in-water emulsions, or alum; CpG can be formulated with alum or with other cationic carriers.

Combinations of adjuvants may be utilized in the present invention, particularly combinations of monophosphoryl lipid A and saponin derivatives, more particularly combinations of QS21 and 3D-MPL, or compositions in which QS21 is quenched in cholesterol-containing liposomes (DQ). Alternatively, the combination of CpG + saponins, such as QS21, are suitable adjuvants for use in the present invention, as are effective adjuvant formulations comprising QS21, 3D-MPL and tocopherol in an oil-in-water emulsion. Saponin adjuvants can be formulated in liposomes and combined with immunostimulatory oligonucleotides. Thus, suitable adjuvant systems include, for example, a combination of monophosphoryl lipid A (preferably 3D-MPL) together with an aluminum salt. Another exemplary adjuvant comprises QS21 and/or MPL and/or CpG. QS21 can be quenched in liposomes containing cholesterol.

The present invention also provides a method for preparing the immunogenic composition or vaccine of the present invention, comprising the step of mixing the AβO*3F of the present invention with a pharmaceutically acceptable excipient or carrier.

The composition of the present invention may be in aqueous form (i.e., solution or suspension) or in dry form (e.g., freeze-dried). If a dry vaccine is used, it is reconstituted into a liquid medium prior to injection. Freeze-drying of vaccines is known in the art. When the immunogenic composition of the present invention includes freeze-dried components, the components are usually prepared separately, mixed and then freeze-dried. In order to stabilize the antigen during freeze-drying, inactive components may be added prior to freeze-drying, for example as stabilizer. Preferred stabilizers to be included are lactose, sucrose and mannitol, and mixtures thereof, such as lactose/sucrose mixtures, sucrose/mannitol mixtures, etc. The final vaccine obtained by aqueous reconstitution of the freeze-dried material may therefore contain lactose and/or sucrose. Preferably, amorphous excipients and/or amorphous buffers are used when preparing freeze-dried vaccines.

In certain embodiments, the composition described herein is formulated to be suitable for the intended route of administration to a subject. For example, the composition described herein (e.g., pharmaceutical and/or immunogenic composition) can be formulated for subcutaneous, parenteral, oral, sublingual, buccal, intradermal, transdermal, colorectal, intraperitoneal, rectal administration, intravenous, intranasal, intratracheal, intramuscular, topical, transdermal or intradermal administration. In a specific embodiment, the composition provided herein (e.g., pharmaceutical and/or immunogenic composition) is formulated for intramuscular injection. In some embodiments, the Aβo*3F described herein can be used to prevent and/or treat AD, for example, as a vaccine for inducing an immune response. As used herein, induction of an immune response refers to the ability of the Aβo*3F (also referred to as an "antigen" or "immunogen") to induce T cell and/or humoral immune response against the Aβo*3F. For example, an immunogenic composition can induce a population of memory T and/or B cells relative to an untreated subject after immunization with the composition.

Immune response may be measured by methods known in the art, including assays for proliferation of specific target lymphocyte types (e.g., B cells, T-cells, T cell lines, and T cell clones) or induction of effector function.

Thus, in some embodiments, a method of inducing an immune response in a subject is provided, comprising administering the Aβo*3F or the immunogenic composition of the present invention to the subject. The immune response is preferably protective and preferably involves antibodies. The method may produce a boosting response. The composition of the present invention is preferably administered to the patient in a 0.5 ml dose (as discussed above). In one embodiment, the subject is the Aβo*3F seronegative. If the subject has no serological evidence of past or current presence of the Aβo*3F, the subject is "seronegative". In another embodiment, the subject is the Aβo*3F seropositive. If the subject has serological evidence of past or current presence of the Aβo*3F, the subject is "serologically positive".

Also provided is an administration regimen designed to maximize the immunogenicity of the Aβo*3F or the immunogenic composition of the present invention. Thus, in one embodiment, a method of inducing an immune response in a subject is provided, comprising administering two or more doses of the AβO*3F and/or the immunogenic composition of the present invention to the subject. In certain embodiments, the doses are separately administered by one, two, three, four, five, six, seven, eight, nine, ten, eleven, twelve or more weeks. In another embodiment, the doses are separately administered by one, two, three, four, five, six, seven, eight, nine, ten, eleven, twelve or more months. Alternatively, the doses may be separately administered by one, two, three, four, five, six, seven, eight, nine, ten, eleven, twelve or more years.

Also provided is a method of treating and/or preventing AD in a subject, comprising administering the Aβo*3F or the immunogenic composition of the present invention to the subject.

In certain embodiments, the composition or the AβO*3F described herein can be administered to a subject to induce an immune response, which includes antibodies. Such antibodies can be isolated using techniques known to those skilled in the art (e.g., immunoaffinity chromatography, immunoprecipitation, centrifugation, etc.).

The ability of the AβO*3F or the composition described herein to generate an immune response in a subject can be evaluated using any method known to a person skilled in the art or described herein, such as an immunoassay such as an ELISA (see, e.g., Van den Dobbelsteen et al., 2016, Vaccine 34: 4152-4160), or an electrochemiluminescence or chemiluminescence-based immunoassay.

The antibodies induced, triggered or identified using the AβO*3F or the composition provided herein can be used to monitor the efficacy of therapy and/or disease progression. Any immunoassay system known in the art can be used for this purpose, including but not limited to competitive and non-competitive assay systems using the following techniques: such as radioimmunoassay, ELISA (enzyme-linked immunosorbent assay), immunoassay based on electrochemiluminescence or chemiluminescence, "sandwich" immunoassay, precipitation reaction, gel diffusion precipitation reaction, immunodiffusion assay, immunoradiometric assay, fluorescent immunoassay, protein A immunoassay and immunoelectrophoresis assay. Several of these assays, such as immunoassay based on electrochemiluminescence or chemiluminescence, can be performed in multiplex form, and the multiplex assay form is generally preferred.

The present invention also provides the AβO*3F or the composition of the present invention for use as a medicament.

The present invention also provides use of the AβO*3F or the composition of the present invention in the manufacture of a medicament for generating an immune response in a mammal.

These uses and methods are preferably for preventing and/or treating MCI and/or AD in a subject.

In some embodiments, the AβO*3F or the composition of the present invention can be used to diagnose MCI and/or AD in a subject.

As used herein, the term "treatment" refers to therapeutic treatment and preventive measures, wherein the purpose is to prevent or slow down (mitigate) undesirable physiological changes or disorders, such as the development of AD. Beneficial or desired clinical results include, but are not limited to, alleviation of symptoms, reduction in severity of the disease, stabilization of the disease state (i.e., no deterioration), delay or slowing of disease progression, improvement or alleviation and relief (whether partial relief or complete relief) of the disease state, whether detectable or undetectable. "Treatment" can also mean that the survival period is prolonged compared to the expected survival period when not receiving treatment. Those in need of treatment include those who have suffered from the disease or disorder and those who are prone to the disease or disorder or those who want to prevent the manifestation of the disease or disorder. "Drug" as used herein is a medicament for treating undesirable physiological changes or disorders.

The term "antiserum" as used herein refers to a serum containing polyclonal antibodies. In some embodiments, the antiserum is obtained by immunizing a target organism, such as a mouse, rat, guinea pig, rabbit, monkey, goat, sheep, horse, cattle, or other mammal, with the AβO*3F or the composition described herein.

The term "polyclonal antibody" as used herein refers to a mixture of multiple antibodies produced by stimulating the body with multiple antigenic determinants of an antigen, that is, a polyclonal antibody. Since the AβO*3F described herein is a complex protein oligomer, the antibodies produced by immunizing animals with the oligomer are usually polyclonal antibodies. The polyclonal antibodies produced can be separated and purified, such as by affinity chromatography purification, immunoprecipitation, molecular exclusion chromatography, etc., to further obtain antibodies against single antigenic determinant. The term "antibody" as used herein refers to an immunoglobulin molecule or a fragment of an immunoglobulin molecule which has the ability to bind to an epitope of an antigen. Naturally occurring antibodies typically comprise a tetramer which is usually composed of at least two heavy (H) chains and at least two light (L) chains. Immunoglobulins include the following isotypes: IgG, IgA, IgM, IgD, and IgE, and the corresponding heavy chains thereof are µ chain, δ chain, γ chain, α chain, and ε chain, respectively. The same class of Igs can be divided into different subclasses according to the differences in the amino acid compositions of the hinge regions and the numbers and positions of the heavy chain disulfide bonds, such as IgG can be divided into IgG1, IgG2, IgG3, IgG4 subtypes, and IgA can be divided into IgA₁ and IgA₂ subtypes. Light chains can be divided into κ chains and λ chains according to the differences in constant regions. The antibody of the invention may be of any isotype. The choice of isotype typically will be guided by the desired effector functions, such as ADCC induction. Exemplary isotypes are IgG1, IgG2, IgG3, and IgG4. Either of the human light chain constant domains, κ or λ, may be used. If desired, the class of the antibody of the present invention may be switched by known methods. For example, an antibody of the present invention that was originally IgG may be class switched to an IgM antibody of the present invention. Further, class switching techniques may be used to convert one IgG subclass to another, for instance from IgGl to IgG2. Thus, the effector function of the antibodies of the present invention may be changed by isotype switching to, e.g., an IgG1, IgG2, IgG3, IgG4, IgD, IgA, IgE, or IgM antibody for various therapeutic uses, provided that the C1q binding activity of the antibody is reduced or eliminated. In some embodiments, the antibody of the present invention is an IgM or an antibody of IgG1, 2, 3 or 4 type. An antibody is said to be of a particular isotype if its amino acid sequence is most homologous to that isotype, relative to other isotypes.

Herein, the term "antibody" is used in the broadest sense thereof, and refers to a protein comprising an antigen-binding site, encompassing natural and artificial antibodies of various structures, including but not limited to intact antibodies and antigen-binding fragments of antibodies.

A "variable region" or "variable domain" is a domain of a heavy or light chain of an antibody involving in the binding of the antibody to its antigen. Each heavy chain of an antibody consists of a heavy chain variable region (herein referred to as VH) and a heavy chain constant region (herein referred to as CH), wherein the heavy chain constant region usually consists of 3 domains (CH1, CH2 and CH3). Each light chain consists of a light chain variable region (herein referred to as VL) and a light chain constant region (herein referred to as CL). The variable regions of the heavy and light chains are typically responsible for antigen recognition, while the constant domains of the heavy and light chains can mediate the binding of the immunoglobulin with host tissues or factors (including various cells of the immune system (e.g., effector cells), Fc receptors and the first component of the classical complement system (C1q)). The heavy and light chain variable regions comprise binding regions interacting with an antigen. The VH and VL regions can be further subdivided into hypervariable regions (HVRs) called "complementarity determining regions (CDRs)", which are interspersed with more conserved regions called "framework regions (FRs)". Each VH or VL consists of three CDR domains and four FR domains, arranged from the amino-terminus to the carboxy-terminus in the following order: FR1 - CDR1 - FR2 - CDR2 - FR3 - CDR3 - FR4.

The terms "complementarity determining region" or "CDR region" or "CDR" (used interchangeably herein with the hypervariable region "HVR") refer to a region in the antibody variable domains which is hypervariable in sequence and forms a loop structurally determined ("a hypervariable loop") and/or comprises antigen-contacting residues ("antigen-contacting points"). The CDRs are primarily responsible for binding to an epitope. Herein, the three CDRs of the heavy chain are referred to as HCDR1, HCDR2 and HCDR3, and the three CDRs of the light chain are referred to as LCDR1, LCDR2 and LCDR3.

It should be noted that the boundaries of the CDRs of the variable regions of the same antibody obtained based on different numbering systems may be different from each other. That is, the CDR sequences of the variable regions of the same antibody defined under different numbering systems are somewhat different from each other. Thus, when defining an antibody with the particular CDR sequences as defined in the invention, the scope of said antibody also covers antibodies of which the variable region sequences comprise said particular CDR sequences, but due to the different numbering system(s) used (such as different numbering system rules or combinations thereof), the CDR boundaries claimed by them might be different from the specific CDR boundaries defined in the invention.

The terms "mAbs", "monoclonal antibodies" or "monoclonal antibody composition" as used herein refer to a preparation of antibody molecules of single molecular composition, and refer to antibodies obtained from a substantially homogeneous population of antibodies, i.e., the population comprising individual antibodies are identical except for the naturally occurring mutations that may be present in minor amounts. A conventional monoclonal antibody composition displays a single binding specificity and affinity for a particular epitope. In certain embodiments, a monoclonal antibody can be composed of more than one Fab domain thereby increasing the specificity to more than one target. The term "monoclonal antibody" or "monoclonal antibody composition" is not limited by any particular method of production (e.g., recombinant, transgenic, hybridoma, etc.).

The present invention also includes "bispecific antibodies".

The term "antigen-binding fragment of antibody" refers to a fragment, a part, a region or a domain of an antibody capable of binding to an epitope (e.g., obtainable by cleavage, recombination, synthesis, etc.). An antigen-binding fragment may comprise 1, 2, 3, 4, 5 or all 6 CDR domains of such antibodies and, while capable of binding to the epitope, it may exhibit different specificities, affinities or selectivities. Preferably, an antigen-binding fragment comprises all 6 CDR domains of said antibody. An antigen-binding fragment of an antibody may be a part of a single polypeptide chain (e.g., scFv) or comprise a single polypeptide chain, or may be a part of two or more polypeptide chains (each having an amino- and carboxy-terminus) (e.g., a bispecific antibody, a Fab fragment, a Fab' fragment, a F(ab')₂ fragment, a Fd fragment, a Fv fragment, a dAb fragment, a camel antibody or a nanobody etc.).

In some embodiments, the antibodies and antigen-binding fragments thereof of the present invention are single chain antibodies.

In some embodiments, the antibodies and antigen-binding fragments thereof of the present invention are chimeric antibodies.

In some embodiments, the antibodies and antigen-binding fragments thereof of the present invention are humanized antibodies.

In some embodiments, the antibodies and antigen-binding fragments thereof of the present invention are human antibodies or fully human antibodies.

Variant antibodies are also within the scope of the present invention.

The numbering of amino acid residues in the invention is according to IMGT^{®}, the international ImMunoGeneTics information system^{®} or Kabat, E. A., Wu, T. T., Perry, H. M., Gottesmann, K. S. & Foeller, C. (1991). Sequences of Proteins of Immunological Interest, 5th edit., NIH Publication no. 91-3242 U.S. Department of Health and Human Services; Chothia, C. & Lesk, A. M. (1987). Canonical Structures For The Hypervariable domains Of Immunoglobulins. J. Mol. Biol. 196, 901-917. Unless otherwise specified, the numbering of amino acid residues in the invention is according to the Kabat numbering system.

An antibody or an antigen-binding fragment thereof is said to "specifically" bind a region of another molecule (*i.e.,* an epitope) if it reacts or associates more frequently, more rapidly, with greater duration and/or with greater affinity or avidity with that epitope relative to alternative epitopes. In some embodiments, the antibody or antigen-binding fragment thereof of the invention binds to a human amyloid protein, particularly its toxic form, particularly the AβO*3F, with an affinity of at least 10⁻⁷ M, such as 10⁻⁸ M, 10⁻⁹ M, 10⁻¹⁰ M, 10⁻¹¹ M or higher. Preferably, the antibody or antigen-binding fragment thereof binds under physiological conditions (e.g., *in vivo*). Therefore, specifically binding to an amyloid protein, particularly its toxic form (particularly the AβO*3F), refers to the ability of the antibody or antigen-binding fragment thereof to bind to an amyloid protein, particularly its toxic form (particularly the AβO*3F), with the above-mentioned specificity and/or under such conditions. Methods suitable for determining such a binding are known in the art.

The term "binding" in the context of the binding of an antibody to a predetermined antigen typically refers to binding with an affinity corresponding to a KD of about 10⁻⁶ M or less, which is at least ten-fold lower, such as at least 100-fold lower, for instance at least 1,000-fold lower than its affinity for binding to a non-specific antigen (e.g., BSA, casein) other than the predetermined antigen or a closely-related antigen.

As used herein, the term "kd" (sec -1 or 1/s) refers to the dissociation constant of a particular antibody-antigen interaction. Said value is also referred as the k_{off} value.

As used herein, the term "ka" (M-1 x sec-1 or 1/Msec) refers to the association rate constant of a particular antibody-antigen interaction.

As used herein, the term "KD" (M) refers to the dissociation equilibrium constant of a particular antibody-antigen interaction and is obtained by dividing the kd by the ka.

As used herein, the term "KA" (M-1 or 1/M) refers to the association equilibrium constant of a particular antibody-antigen interaction and is obtained by dividing the ka by the kd.

Antibodies of the invention are produced by any technique known in the art, such as, but not limited to, any chemical, biological, genetic, or enzymatic technique, alone or in combination. In general, if knowing the amino acid sequence of a desired sequence, one skilled in the art can readily generate such an antibody by standard techniques used to generate polypeptides. For example, these antibodies can be synthesized using well-known solid-phase methods, preferably using a commercially available peptide synthesis apparatus (such as that manufactured by Applied Biosystems, Foster City, California) and following the manufacturer's instructions. Alternatively, antibodies of the invention can be synthesized by recombinant DNA techniques well known in the art. For example, after incorporating a DNA sequence encoding an antibody into an expression vector and introducing these vectors into eukaryotic or prokaryotic host cells suitable for expressing the desired antibodies, antibodies as DNA expression products can be obtained, which can then be isolated from the host cells using known techniques.

The antibodies and antigen-binding fragments thereof of the present invention can be modified by including any "suitable" number of modified amino acids and/or in combination with coupling substituents.

The antibodies and antigen-binding fragments thereof of the present invention can also be chemically modified by covalent coupling to a polymer to increase its circulation half-life.

Antibodies and antigen-binding fragments thereof of the invention can be produced in different cell lines, such as human cell lines, non-human mammalian cell lines, and insect cell lines, such as CHO cell lines, HEK cell lines, BHK-21 cell lines, murine cell lines (such as myeloma cell line), fibrosarcoma cell line, PER.C6 cell line, HKB-11 cell line, CAP cell line, and HuH-7 human cell line (Dumont et al., 2015, Crit Rev Biotechnol., Sep. 18, 1-13., the contents of which are incorporated herein by reference).

The antibodies of the invention are suitably separated from the culture medium by conventional immunoglobulin purification methods, such as Protein A-Sepharose, hydroxyapatite chromatography, gel electrophoresis, dialysis, or affinity chromatography.

The term "subject" refers to warm-blooded animal, preferably, mammal (including human, livestock and farm animal, zoo animal, sports animal or pet animal, such as dog, cat, cattle, horse, sheep, pig, goat, rabbit, etc.), more preferably, human. In one embodiment, the subject can be a "patient", that is, warm-blooded animal, more preferably, human, who is waiting to receive or is receiving medical care or will be the subject of medical procedure or disease development monitoring. In one embodiment, the subject is an adult (e.g., subject over 18 years old). In another embodiment, the subject is a child (e.g., subject under 18 years old). In one embodiment, the subject is an elderly person who is older than 55, 60, 65, 70, 75, 80, 85, 90 years old or older. In one embodiment, the subject is a male. In another embodiment, the subject is a female.

The technical solutions of the present invention will be specifically described below by examples, which are descriptive and illustrative, and are not meant to be limiting. The reagents used in the following examples, if not specifically noted, can be easily purchased from reagent companies such as Sigma Aldrich and Merck, and the test methods, if not specifically noted, can be found in textbooks such as Sambrook, J., Fritsch, EF and Maniatis, T. (1989) Molecular Cloning: A Laboratory Manual. Cold Spring Harbor Press, New York.

### Examples

### Example 1 Obtaining of Aβ oligomers specifically bound by the antibody 3F

### 1.1 Experimental Materials and Methods

### 1.1.1 Experimental Materials

Protein A Magnetic Beads: Bio-RAD, #1614833
Protein G Magnetic Beads: Bio-RAD, #1614023
Protein A/G Agarose Gel: Abmart, #A10001S

The antibody 6E10: Biolegend, #803002
Human Aβ42 detection kit: Immuno-Biological Laboratories

### 1.1.2 Main solution

(1) 50 mM sodium hydroxide (pH 10.0);
(2) 20 mM phosphate buffer (PBS): pH 7.4;
(3) 0.1% PBST: PBS containing 0.1% Tween-20;
(4) 20 mM glycine elution buffer (pH 2.0);
(5) 1 M Tris neutralization solution: pH 10.0;

Note: The relevant reagents and solutions mentioned in Examples 2, 3, 4, 5 and 6 with no specified source are the same as those in Example 1.

### 1.2 Preparation of mouse brain homogenate

APP/PS1 mice were deeply anesthetized with sodium pentobarbital and sacrificed after transcardial perfusion with ice-cold PBS containing heparin (10 U/mL). 1 mL of RIPA strong lysis reagent (containing protease inhibitors and phosphatase inhibitors) was added to the brain tissue and lysed using Tissue Lyser II tissue homogenizer at a frequency of 30 Hz for 8 minutes, centrifuged at 4 °C, 14,000 rpm for 30 minutes, and collect the supernatant. The brain homogenate was added to 100 µ L of Protein A/G-agarose gel before immunoprecipitation, and incubated at 4 °C for 1 h to remove endogenous IgG in mouse brain homogenate. Then, the brain homogenate was added to Protein A magnetic beads cross-linked with APP antibody to remove endogenous APP in mouse brain.

### 1.3 Cross-linking antibodies with magnetic beads

First, the antibodies 3F and 6E10 were cross-linked with Protein A or Protein G magnetic beads, respectively. The specific cross-linking steps were as follows:
(1) Take 200 µL of Protein A or Protein G magnetic beads and wash them three times with 2 mL of 0.1% PBST. Mix the beads thoroughly each time and place on a magnetic rack. Discard the supernatant.
(2) Add 50 µg of the antibody 3F or 6E10 to the magnetic beads, shake at room temperature for 30 min to allow the beads to fully bind to the antibody, then wash three times with 0.1% PBST;
(3) Wash the magnetic beads with cross-linking buffer (0.2 M triethanolamine, pH 8.2) once, then dissolve 12 mg of DMP in 2 mL of cross-linking buffer, add to the magnetic beads, and shake at room temperature for 1 h. After the reaction is completed, discard the cross-linking solution, add 2 mL of blocking buffer (0.1 M ethanolamine, pH 8.2) to wash once, and then add 2 mL of blocking buffer again, and block at room temperature for 2 h;
(4) After blocking, wash the magnetic beads three times with PBS, then elute them once with 0.1 M glycine (pH 2.5), react for 5 min, and then wash the magnetic beads three times with 0.1% PBST. Immerse the magnetic beads in PBST containing 0.02% NaN3 and store them at 4 °C.

### 1.4 In vitro preparation of the Aβo*3F

1 mg of Aβ42, Aβ40 or other forms of Aβ (Chinese Peptide Co., Ltd.) was dissolved in 1 mL of 100% hexafluoroisopropanol (HFIP), vortexed for 5 min, and ultrasonicated in a water bath for 10 min, then dispensed into EP tubes, the solvent was evaporated overnight, and stored at -20 °C. Before use, the HFIP-treated and aliquoted Aβ was dissolved in 50 mM NaOH at a concentration of 1 mg/mL, vortexed for 3-5 min, ultrasonicated for 1 min, and then diluted to 10 µM with pre-cooled PBS. Centrifuged at 21,000 g for 30-40 min at 4 °C, discarded the precipitate (about 5% of the starting volume), and Aβ monomers were obtained. The Aβ monomers were statically incubated at 25 °C for 2 days, and then incubated with Protein A magnetic beads cross-linked with the antibody 3F at 4 °C overnight. On the next day, the magnetic beads were washed three times with 0.1% PBST and eluted twice with 20-100 mM glycine (pH 2.0) for 3-5 min. The eluate was neutralized to pH7 with 1 M Tris to obtain sAβo*3F (Aβos prepared *in vitro*).

### 1.5 Isolation and preparation of the Aβo*3F from APP/PS1 mouse brain homogenate and AD patient CSF

To isolate and prepare Aβos specifically recognized by the antibody 3F (Aβo*3F), brain homogenates of APP/PS1 mice and CSF samples of AD patients (obtained from the First Affiliated Hospital of Zhengzhou University, with informed consent and approval from the Ethics Review Committee of the First Affiliated Hospital of Zhengzhou University) were incubated with Protein A magnetic beads cross-linked with the antibody 3F at 4°C overnight. The next day, the magnetic beads were washed three times with 0.1% PBST and then eluted twice with 20 mM-100 100 mM glycine (specifically 20 mM, pH2.0)for 3 min, and the eluate was neutralized to pH7 with 1 M Tris, and then the Aβo*3F in the brains of APP/PS1 mice (mAβo*3F, the Aβo*3F isolated from the brains of AD mice) or the Aβo*3F extracted from the CSF of AD patients (hAβo*3F, the Aβo*3F isolated from the cerebrospinal fluid of AD patients) was obtained. The Aβ aggregate mixture after the antibody 3F immunodepletion is called Aβ-ID, which are sAβ- ID (prepared *in vitro*), mAβ- ID (isolated and prepared from the brains of APP/PS1 mice) and hAβ- ID (isolated and prepared from the CSF of AD patients) and used as controls.

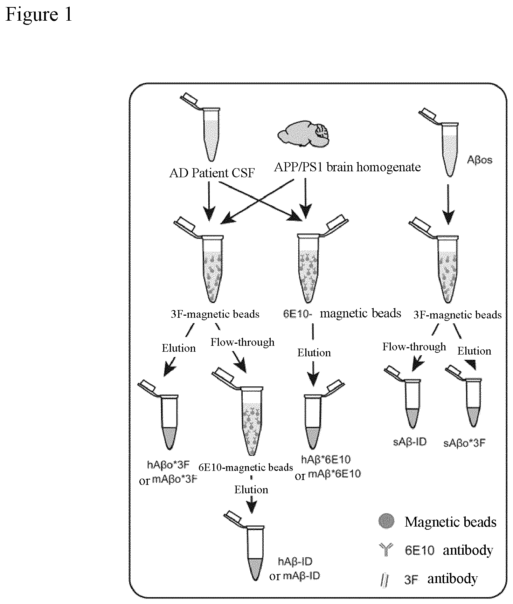

The Aβ aggregate mixture Aβ*6E10 was prepared by mixing APP/PS1 mouse brain homogenate or AD patient CSF with Protein G magnetic beads cross-linked with the 6E10 antibody, reacting at 4°C overnight, and then eluting twice with 20 mM-100 mM glycine (specifically 20 mM, pH 2.0) for 3-5 min (specifically 3 min), and the eluate was neutralized to pH7 with 1 M Tris, and then Aβo*6E10 in the brain of APP/PS1 mice (mAβo*6E10, Aβo*6E10 isolated from the brains of AD mice) or Aβo*6E10 extracted from the CSF of AD patients (hAβo*6E10, Aβo*6E10 isolated from the CSF of AD patients) was obtained (Figure 1). The concentration of Aβ obtained by immunoprecipitation was measured using an Aβ detection kit.

### Example 2 Characterization of molecular weight and morphology of the Aβo*3F

### 2.1 Experimental Materials and Methods

### 2.1.1 Experimental Materials

Aβ polypeptide: Chinese Peptide Co., Ltd.;
Superdex 200 10/300 GL molecular exclusion chromatography: GE Healthcare;
The antibody A11: Invitrogen, #AHB0052;
Molecular exclusion chromatography protein marker: GE healthcare;
3-8% Tris-Acetate precast gel: Invitrogen;
Carbon film support copper mesh: Zhongke Keyi;
Uranyl acetate: Zhongke Keyi;
ECL chemiluminescence kit: Pierce Biotechnology;
ThT detection reagent: Sigma-Aldrich;
Nitrocellulose membrane: Millipore.
2.1.2 Experimental instruments
AKTA protein chromatograph: GE Healthcare Life Science, USA;
Protein electrophoresis instrument: Bio-rad, USA;
Protein transfer system: Bio-rad, USA;
HT7700 transmission electron microscope: Hitachi, Japan;
Safire2^{™} microplate reader: Tecan Group, Switzerland;
Amersham Imager 680 imaging system: GE, USA.

### 2.1.3 Main solutions

(1) 20 mM TBS buffer: Weigh 2.4 g Tris and 8 g NaCl, dissolve in deionized water, adjust its pH to 7.5 with dilute hydrochloric acid, and make up to 1 L.
(2) 0.1% TBST: Add 1 mL of Tween-20 to 1 L of TBS and stir for 20 min.
(3) ThT solution: Prepare 100X ThT stock solution (500 µM) with 50 mM PB (phosphate buffer, pH = 6.5) solution. Dilute to 1X with 50 mM PB (pH = 6.5) before use.
(4) 5% skim milk.
(5) Electrophoresis buffer: Dissolve 30 g Tris, 144 g Glycine, and 10 g SDS, make up to 1 L with deionized water, and dilute 10-fold before use.
(6) 5x non-reduced loading buffer (10 mL): 2 mL 10% SDS, 0.6 mL 1 M Tris-HCl (pH 6.8), 5 mL glycerol, 1 mL 1% bromophenol blue, and make up to 10 mL with deionized water.
(7) Transfer buffer: Dissolve 30 g Tris and 144 g Glycine in deionized water and make up to 1 L. Dilute 10-fold before use.

### 2.2 Preparation and characterization of Aβ monomers, oligomers, and fibers

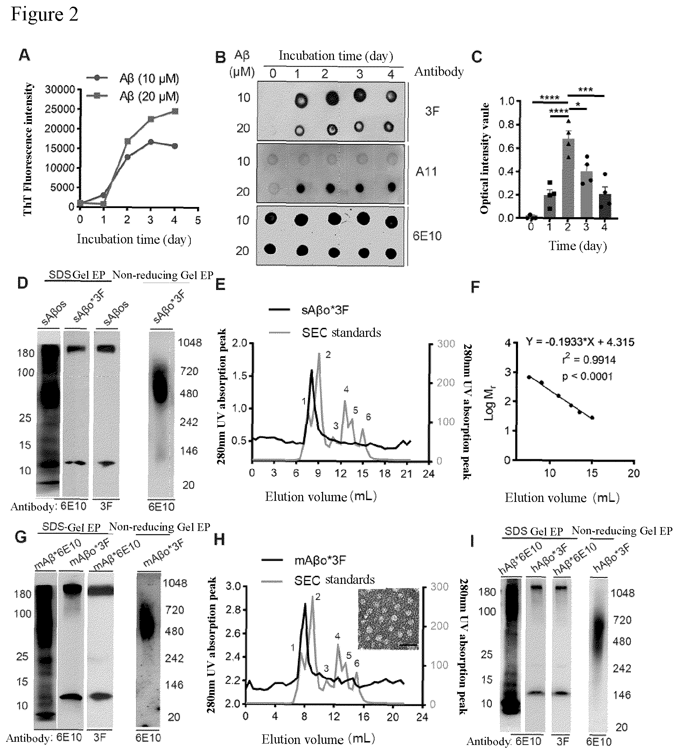

The preparation of Aβ monomers was the same as in Item 1.4 above. Aβ monomers were incubated at 25°C for 0-4 days, and the aggregation state of Aβ was detected by ThT every 24 h. The specific procedure was as follows: Aβ sample was diluted to 10 µM with PBS buffer, and then 190 µL of ThT detection solution and 10 µL of the sample to be tested were thoroughly mixed in a black plate, and the fluorescence was measured using a Safire2 ^{™} plate reader with an excitation wavelength of 440 nm and an emission wavelength of 480 nm. The results showed that as the incubation time increased, the ThT reading of the Aβ sample gradually increased, indicating that the Aβ monomers gradually aggregated into oligomers and fibers (Figure 2, panel A).

Dot blot experiments were used to evaluate the binding of different antibodies to Aβ monomers and aggregates. Aβ samples were spotted on nitrocellulose membranes and blocked with 5% skim milk for 1 h at room temperature. The membranes were incubated with different detecting antibodies for 1-2 h at room temperature, then washed three times with 0.1% TBST for 5 min each time, and HRP-labeled secondary antibodies were added for incubation at room temperature for 1 h. The membranes were washed three times with 0.1% TBST for 5 min each time, and ECL chemiluminescent solution was added to the membranes. The Amersham Imager 680 imaging system was used for color development, and the size and optical density of the blots were quantitatively analyzed using Image J software. The results showed that the antibody 6E10 is an antibody that binds widely to Aβ monomers, oligomers, and fibers. It can bind to 10 µM and 20 µM Aβ, and there is no significant difference in binding. Neither the antibody 3F nor A11 binds to Aβ monomers, and the antibodies 3F and A11 bind to different oligomer types. The antibody 3F mainly binds to 10 µM Aβos, while the antibody A11 mainly binds to 20 µM Aβos, indicating that the antibodies 3F and A11 recognize different oligomer conformations (Figure 2, panel B). Additionally, the affinity between 10 µM Aβos and the antibody 3F obtained by incubation for 0-4 days showed that the 10 µM Aβos (sAβos) obtained by 2-day incubation had the highest affinity with the antibody 3F. Therefore, the subsequent experiments used this oligomer (sAβo*3F, the Aβo*3F prepared *in vitro*) and the antibody 3F for immunoprecipitation (Figure 2, panel C).

### 2.3 Characterization of molecular weight of sAβo*3F

The molecular weight of sAβo*3F was detected by Western blot. The specific procedure was as follows: Aβ samples were loaded onto 15% SDS-PAGE gel electrophoresis or 3-8% Tris-Acetate gel. For Tris-Acetate gel, the sample was mixed with Novex^{™} Tris-Glycine sample buffer before loading. Electrophoresis was performed in Tris-Glycine electrophoresis buffer (containing 0.5 mM DTT, 1 mM ATP and 5 mM MgCl2) at 4 °C, 150 V for 4 h. For Western blot, proteins separated by SDS-PAGE or Tris-Acetate gel were transferred onto nitrocellulose membrane. The membrane was blocked with 5% skim milk for 1-2 h at room temperature, incubated with the detection antibody (6E10) at 4 °C overnight, and then washed three times with 0.1% TBST for 5 min each time, and reacted with the corresponding secondary antibody (HRP-labeled goat anti-mouse secondary antibody) for 1 h at room temperature. After washing three times with TBST, the membrane was covered with ECL developing solution, developed in an Amersham Imager 680 imaging system, and the protein bands were quantified using Image J software.

The SDS-PAGE experiment results showed that sAβo*3F was mainly composed of oligomers with two different molecular weights, one was approximately 12 kDa and the other was greater than 180 kDa. The results of native-PAGE showed that sAβo*3F was mainly composed of an oligomer with a molecular weight of approximately 500 kDa. The difference between the two electrophoresis results may be due to the influence of SDS (Figure 2, panel D).

In order to further determine the molecular weight of sAβo*3F, 500 µL sAβo*3F or 100 µL SEC Marker was loaded onto a Superdex 200 10/300 GL molecular exclusion chromatography column connected to an AKTA pure system. The column was pre-equilibrated with Tris-Gly buffer and eluted at a flow rate of 0.5 mL/min. By comparing with the Marker, the molecular weight of sAβo*3F was found to be 588 kDa (Figure 2, panels E and F).

### 2.4 Characterization of molecular weight and morphology of mAβo*3F

The Aβo*3F in the brain of APP/PS1 mice was extracted (mAβo*3F) and its molecular weight was analyzed. Consistent with the results of sAβo*3F, mAβo*3F showed two bands in SDS-PAGE, with molecular weights of 12 kDa and greater than 180 kDa, respectively. While in native-PAGE, mAβo*3F had only one band with a molecular weight of about 500 kDa (Figure 2, panel G). SEC results showed that the molecular weight of mAβo*3F was 588 kDa (Figure 2, panel H). In addition, 10 µL of the Aβo*3F was dropped on a 200-mesh copper grid and adsorbed for 20 min, then dried with filter paper, negatively stained with 2% uranyl acetate for 30s, dried with filter paper, and air-dried. The samples were examined under a transmission electron microscope (TEM, Hitachi H7700, Japan) at a magnification of 100,000× with 120 kV operating voltage. The results showed that mAβo*3F were particles with a diameter of about 10 nm (Figure 2, panel H).

### 2.5 Characterization of molecular weight of Aβo*3F in CSF of AD patients

The molecular weight and distribution of the Aβo*3F extracted from CSF of AD patients (hAβo*3F) were consistent with the results of sAβo*3F and mAβo*3F. hAβo*3F had only one band in native-PAGE with a molecular weight of approximately 500 kDa, while in SDS-PAGE there were two bands with molecular weights of 12 kDa and greater than 180 kDa, respectively (Figure 2, panel I).

### Example 3 Neurotoxicity Detection of the Aβo*3F

### 3.1 Experimental Materials

N2a cells: National Laboratory Cell Resource Sharing Service Platform (NICR);
D-Poly-lysine hydrobromide (PDL): Sigma-Aldrich
B27 Supplement (50x): Thermo Fisher Scientific
Neurobasal^{™}-A Medium: Gibco
70 µm nylon cell strainer: BD Bioscience
3.2 Primary neuron culture

The fetal brain of C57BL/6 (purchased from Beijing HFK Bioscience Co., Ltd.) at 14 to 15 days of gestation was removed, the blood and brain membranes were stripped in HBSS (Hank's balanced salt solution), and the separated hippocampus and cortex were cut into pieces with forceps. The broken tissue pieces were transferred to a 15 mL centrifuge tube and centrifuged at 600 rpm for 3 min. 10 mL of 10-fold diluted trypsin digestion solution (containing DNase I ) was added to the precipitate and digested at 37 °C for 10 min, gently inverted several times every 5 min. After digestion, 40 mL of DMEM medium (containing 10% FBS) was used to terminate the trypsin digestion reaction, and the cell suspension was passed through a 70 µm cell sieve to remove undigested tissue pieces. The cells were collected by centrifugation at 1,200 rpm for 10 min, and the cells were resuspended in DMEM medium and plated in a 12-well plate. After 2-4 h, the medium was changed to Neurobasal medium (containing B27, 0.5 mM L-glutamine, 0.5% penicillin and streptomycin) and cultured for 7-9 days for subsequent experiments.

### 3.3 Cytotoxicity detection of sAβo*3F

This experiment mainly used the MTT assay to detect the toxicity of Aβ aggregates to neurons. The specific procedure was as follows: N2a cells were cultured in DMEM medium containing 10% FBS and 1% penicillin/streptomycin. The cells in the culture dish were digested with trypsin, centrifuged, and resuspended in the medium, and then inoculated in a 96-well plate, with about 5,000 cells/100 µL medium per well. After 12 hours, the cells were treated with a series of concentration gradients of sAβo*3F and sAβos, and the same volume of solvent was added to the cells as a control. After 72 hours, 25 µL 5 mg/mL MTT was added to each well. After further culture at 37 °C for 3 hours, the medium was aspirated and 150 µL DMSO was added. The absorbance at 570 nm and 630 nm was measured using an MD-M5 microplate reader. The average value of six replicate wells was used for each sample and control, and each experiment was repeated three times. Cell viability was calculated by dividing the absorbance (background corrected) of the sample well with the absorbance (background corrected) of the well added with solvent.

MTT results showed that the half-inhibitory concentration (IC50) of sAβo*3F on cell viability was about 0.186 nM, while the IC50 of sAβos before immunoprecipitation was about 63.26 nM, there was a 340-fold difference between the two IC50s (Figure 3, panels A and B). In addition, adding 0.5 nM sAβo*3F into N2a cells can cause 66% cell death, while sAβos and sAβ-ID at the same concentration had no substantial neurotoxic effect. When the concentration of sAβos reaches 200 nM, it can produce 70% cytotoxicity, while the neurotoxicity of sAβ-ID can only reach 32% at this concentration (Figure 3, panel C). These results showed that sAβo*3F is a highly neurotoxic oligomer.

### 3.4 Cytotoxicity detection of mAβo*3F

MTT assay showed that the IC50 of mAβo*3F for N2a cells was about 0.111 nM; the IC50 for primary neuronal cells was about 0.057 nM (Figure 3, panel D). Specifically, 0.5 nM mAβo*3F can cause 78% primary neuronal cell death, while the same concentrations of mAβ*6E10 and mAβ-ID had no substantial cytotoxicity. When the concentration was increased to 200 nM, mAβ*6E10 produced 54% neurotoxicity, while the neurotoxicity of mAβ-ID was only 20% (Figure 3, panel E).

### 3.5 Cytotoxicity detection of hAβo*3F

The IC50 of hAβo*3F on primary neurons detected by MTT was approximately 0.076 nM (Figure 3, panel F). Specifically, adding 0.2 nM hAβo*3F to primary neurons can reduce cell viability by 68%, while hAβ*6E10 and hAβ-ID had no significant effect on neuronal survival at this concentration. When the concentration increased to 40 nM, hAβ*6E10 reduced cell viability by 66%, while hAβ-ID only reduced cell viability by 40% (Figure 3, panel G).

### Example 4 Effect of the Aβo*3F on cytokine expression levels in glial cells

### 4.1 Experimental Materials and Methods

### 4.1.1 Experimental Materials

BV2 cells: NICR
Reverse transcription kit: Kangwei Century
UltraSYBR Mixture (Low ROX): Kangwei Century

### 4.1.2 Experimental instruments

7500 Fast Real-time PCR Instrument: Applied Biosystems
T100 Thermal Cycler PCR instrument: BIO-RAD
NanoDrop Microvolume Spectrophotometer: Quawell

### 4.2 The Aβo*3F can increase the expression level of inflammatory factors in microglia

BV2 cells were cultured in DMEM medium containing 10% FBS and 1% penicillin/streptomycin. The cells in the culture dish were digested with trypsin, centrifuged, resuspended in the medium, and inoculated in a 12-well plate, with approximately 5×10^5 cells/mL medium per well. After 12 h, BV2 cells were treated with different Aβ samples, and the same volume of solvent was added to the cells as a control. After 48 h, the expression level of intracellular proinflammatory factors was detected by fluorescence quantitative PCR (qPCR). The specific procedure was as follows: Total cell mRNA was obtained by conventional methods and the mRNA concentration was determined using a microtube photometer. Reverse transcription was performed according to the instructions of the reverse transcription kit to obtain cDNA, and then the expression level of the target gene was detected using EasyQuick RT MasterMix. The primers used were as follows:

| | |
|---|---|
| mTNF- α : | 5'-GATTATGGCTCAGGGTCCAA-3' (SEQ ID NO: 1), |
| | 5'-GCTCCAGTGAATTCGGAAAG-3' (SEQ ID NO: 2); |
| mIL-1 β : | 5'-CCCAAGCAATACCCAAAGAA-3' (SEQ ID NO: 3), |
| | 5'-GCTTGTGCTCTGCTTGTGAG-3' (SEQ ID NO: 4); |
| mIL-6: | 5'-CCGGAGAGGAGACTTCACAG-3' (SEQ ID NO: 5), |
| | 5'-TTGCCATTGCACAACTCTTT-3' (SEQ ID NO: 6); |
| GAPDH: | 5'-TGAATACGGCTACAGCAACA-3' (SEQ ID NO: 7), |
| | 5'-AGGCCCCTCCTGTTATTATG-3' (SEQ ID NO: 8). |

The qPCR results showed that 0.3 nM mAβo*3F, 200 nM mAβ*6E10 and 200 nM mAβ-ID can induce a significant increase in the expression level of proinflammatory factors in microglia, including TNF-α, IL-6 and IL-1β, but 0.3 nM mAβ*6E10 or 0.3 nM mAβ-ID had no substantial stimulatory effect (Figure 4, panel A).

### 4.3 Primary astrocyte culture

Take 1-2 day old C57BL/6 newborn mice, soak them in 75% alcohol for disinfection, remove the brain and soak in HBSS buffer, remove the blood and brain membrane, cut the brain into pieces with tissue scissors, put it in a 15 mL centrifuge tube, centrifuge at 600 rpm for 3 min, add 10 mL of 10-fold diluted trypsin digestion solution (containing DNase I ) to the precipitate, digest at 37 °C for 10 min, and gently invert a few times every 5 min. After digestion, use 40 mL DMEM medium (containing 10% FBS) to terminate the trypsin digestion reaction, and pass the cell suspension through a 70 µm cell sieve to remove undigested tissue pieces. Centrifuge at 1200 rpm for 10 min to collect cells, resuspend the cells in DMEM medium, and plate the cells in a T-75 culture flask and culture them with DMEM medium at 37 °C for 5-7 days. Change the medium every 2-3 days. After 7 days of cell culture, digest the cells with trypsin and plate them in a 12-well plate for use.

### 4.4 The Aβo*3F can stimulate the expression of inflammatory factors in astrocytes

Primary astrocytes were treated with different Aβ samples, and qPCR was used to explore their effects on the expression levels of inflammatory factors in primary astrocytes. The specific procedure steps were the same as item 4.2 above. The primers used were as follows:
miNos: 5'-CACCTGGAACAGCACTCTCT-3' (SEQ ID NO: 9),
   5'-CTTTGTGCGAAGTGTCAGTG-3' (SEQ ID NO: 10);
mTNF-α: SEQ ID NOs: 1 and 2;
mIL-1β: SEQ ID NOs: 3 and 4;
mIL-6: SEQ ID NOs: 5 and 6;
GAPDH: SEQ ID NOs: 7 and 8.

The results showed that 0.3 nM mAβo*3F, 200 nM mAβ*6E10 and 200 nM mAβ-ID can significantly increase the expression level of proinflammatory cytokines in primary astrocytes, including TNF-α, IL-6, IL-1β and iNos, but 0.3 nM mAβ*6E10 or 0.3 nM mAβ-ID had no significant effect on the expression level of inflammatory factors in astrocytes (Figure 4, panel B).

### 4.5 The Aβo*3F disrupts astrocyte-mediated synaptogenesis.

Astrocytes can induce synapse formation by secreting synaptic factors such as TSP1 and Gpc4/6. mAβo*3F was added to primary astrocytes, and the changes in the expression level of various synaptic factors in astrocytes were detected by qPCR. The primers used were as follows:

| | |
|---|---|
| mGpc4: | 5'-CTGGAGGGTCCTTTCAACATT-3' (SEQ ID NO: 11), |
| | 5'-GACATCAGTAACCAGTCGGTC-3' (SEQ ID NO: 12); |
| mGpc6: | 5'-TAGTCCTGTATTGGCAGCCAC-3' (SEQ ID NO: 13), |
| | 5'-GGCTAATGTCTATAGCAGGGAA-3' (SEQ ID NO: 14); |
| mTSP1: | 5'-GGTAGCTGGAAATGTGGTGCGT-3' (SEQ ID NO: 15), |
| | 5'-GCACCGATGTTCTCCGTTGTGA-3' (SEQ ID NO: 16); |
| GAPDH: | SEQ ID NOs: 7 and 8. |

The results showed treating cells with 0.3 nM mAβo*3F and 200 nM mAβ*6E10 can significantly reduce the expression level of TSP1 and Gpc4/6, while 0.3 nM mAβ*6E10 or 0.3 nM mAβ-ID had no significant effect. Only when the concentration of mAβ-ID was increased to 200 nM, the expression level of Gpc4 in astrocytes was reduced (Figure 4, panel C).

### Example 5 Effects of the Aβo*3F on mouse cognition and damage to brain neurons

### 5.1 Experimental Materials and Methods

### 5.1.1 Experimental Materials

Rapid Golgi Staining Kit: FD NeuroTechnologies
Nissl staining kit: Beijing Solarbio Science & Technology Co.,Ltd.

### 5.1.2 Experimental instruments

Brain stereotaxic injection system: Chinese Academy of Medical Sciences
Y-maze equipment: Chinese Academy of Medical Sciences
Novel object recognition equipment: Chinese Academy of Medical Sciences

### 5.2 Experimental Animals

Three-month-old C57BL/6 mice. All mice were housed in a clean room at 22 ± 2 °C and 45% ± 10% humidity, with free access to food and water, and a 12-h light and 12-h dark cycle. All animal experiments were performed in accordance with the Guidelines for the Care and Use of Laboratory Animals in Public Health Services of China, and experiments involving mice were approved by the Animal Care and Use Committee of Tsinghua University.

### 5.3 Stereotaxic Brain Injection

Three-month-old C57BL/6 mice were randomly divided into 6 groups, 6-8 mice per group, as follows: group injected with 2.5 nM (45.5 pg) mAβo*3F, group injected with 2.5 nM (45.5 pg) mAβ*6E10, group injected with 2.5 nM (45.5 pg) mAβ-ID, group injected with 600 nM (10.9 ng) mAβ*6E10, group injected with 600 nM (10.9 ng) mAβ-ID, and control group injected with Tris-Gly solvent. Mice were deeply anesthetized with a mixture of ketamine (100 mg/kg) and xylazine (10 mg/kg) and fixed on a stereotaxic injection platform. The skin of the mouse brain was cut open along the midline to expose the skull. The injection site was located using a stereotaxic instrument: starting from the bregma, AP = -1.7 mm, ML = ±1.0 mm, and DV = -1.5 mm. After opening the skull with a cranial drill along the positioning point, bilateral injections were performed, with 4 µL injected into each hemisphere at an injection rate of 0.4 mL/min. After the injection, the needle was retained for 3 min to allow the sample to be completely absorbed. The surgical site was washed with sterile saline and the incision was sutured. The status of the mice was monitored and postoperative care was provided. The behavior and cognitive ability of the mice were tested 24 h after the stereotaxic injection, and the mice were dissected for physiological and biochemical analysis.

### 5.4 The Aβo*3F severely impairs the memory ability of mice

The novel object recognition test and Y-maze test were used to detect changes in the cognitive abilities of mice.

### 5.4.1 Novel Object Recognition Test

Novel object recognition test was designed based on the spontaneous tendency of mice to show more interactions with new and unfamiliar objects. The experiment was mainly divided into three stages:
(1) adaptation stage: prepare a white box with a size of 40 cm wide, 40 cm deep, and 40 cm high. Allow each mouse to freely explore the box for 5 min without any objects in it. One hour later, inject the mice with Aβ samples.
(2) training stage: 24 h later, two identical objects were placed in the box, and each mouse was placed in the same box as in the first phase, and allowed to explore freely for 5 min. The number of times the mouse sniffed and touched each object was recorded;
(3) detection stage: after 6 hours of training, the object on the right was replaced with a new object with different material, color, and shape. The mice were allowed to explore freely for another 5 minutes. The number of times the mice sniffed and touched the two objects was recorded to test the memory ability of the mice. The cognitive index was determined by the following calculation formula: (number of times the new object was touched - number of times the old object was touched) / (number of times the new object was touched + number of times the old object was touched).

The results showed that compared with the control group, the cognitive index of mice treated with 2.5 nM mAβo*3F and 600 nM mAβ*6E10 for new objects was significantly decreased, while the cognitive index of mice in other Aβ-treated groups was not significantly different from that of the control group, indicating that the Aβo*3F significantly reduced the memory ability of mice (Figure 5A).

### 5.4.2 Y-maze test

After the novel object recognition test, the spatial memory ability of mice was tested by the Y-maze test. The Y-maze consists of three identical arms with a 120-degree angle between each arm. The size of each arm is 8 cm × 30 cm × 15 cm (width × length × height). The experiment was mainly divided into two stages:
(1) training stage: during the training stage, the three arms were randomly named as A, B, and C. Arms A and B were open throughout the experimental phase, while arm C was a new arm and was closed during the training stage. After being placed in arm A, each mouse was allowed to freely explore arms A and B for 10 min.
(2) after 1 h of training, the C arm was opened and the mice were placed in the A arm in order again. The mice were allowed to explore the A, B, and C arms for 5 min, and the time they stayed in the three arms was analyzed. All the experimental processes were recorded by a camera installed above the Y maze.

The results showed that the duration of mice treated with 2.5 nM mAβo*3F and 600 nM mAβ*6E10 in the new arm was significantly reduced compared with the control mice. However, there was no significant difference between the mice injected with 2.5 nM mAβ*6E10, 2.5 nM mAβ*ID and 600 nM mAβ*ID and the control group. This indicates that mAβo*3F, but not mAβ*6E10 or mAβ-ID, can cause severe memory impairment in mice at low concentrations. For mAβ*6E10, only when the concentration is increased to 600 nM can mice show a similar degree of memory impairment (Figure 5, panel B).

### 5.5 The Aβo*3F significantly reduces the density of dendritic spines in mouse neurons

The mouse brain samples were stained using the FD Fast Golgi Staining Kit. 24 h before dissecting the mice, the A and B solutions were mixed in the kit in a 1:1 ratio and stored in the dark. After the mice were killed, the brain was taken directly without perfusion and divided into left and right hemispheres along the midline. The blood on the surface of the left hemisphere was washed off with PBS, and the solution on the surface of the tissue was soaked off with filter paper, and then the tissue was placed in the pre-prepared A+B solution. The solution was changed once after 24 h and placed at room temperature in the dark for 2 weeks. During the immersion period, the tissue was gently turned upside down twice a week to obtain the best results. After 2 weeks, the tissue was transferred to C solution, the solution was changed once after 24 h, and placed at room temperature in the dark for 5 days. Then, a freezing microtome was used to obtain 100 µm thick frozen sections. After the sections were dried naturally at room temperature, staining began. First, the sections were washed twice with Milli-Q water for 4 min each time, then the staining working solution was prepared, the sections were placed in the staining working solution for 15 min, and washed twice with Milli-Q water for 5 min each time. After staining, the sections were dehydrated with 50%, 75%, 95% and 100% ethanol, and each concentration gradient was processed for 4 min. The sections were then placed in xylene and sealed with neutral resin for observation. The dendrites and dendritic spines of neurons in the CA1 region of the hippocampus were observed using the 100× lens objective of an Olympus inverted microscope. The density of dendritic spines was determined by Image J software analysis (dendritic density = number of dendritic spines/dendritic length).

By calculating the density of dendritic spines, it was found that compared with the control group, the density of dendritic spines in neurons in the CA1 region of the mouse brain treated with 2.5 nM mAβo*3F and 600 nM mAβ*6E10 was significantly decreased, but 2.5 nM mAβ*6E10, 2.5 nM mAβ-ID or 600 nM mAβ-ID had no significant effect on the density of dendritic spines in mouse brain neurons (Figure 5, panels C and D).

### 5.5 The Aβo*3F significantly reduces the number of neurons in mouse hippocampus

### 5.5.1 Paraffin embedding and sectioning

After behavioral experiments, the mice were deeply anesthetized with sodium pentobarbital (50 mg/kg) and a cardiac perfusion was performed with ice-cold PBS containing heparin (10 U/mL), then the mice were killed and dissected. After the brain of the mouse was removed, it was divided into the left and right hemispheres along the midline. The left hemisphere was fixed in 4% paraformaldehyde for 48 hours and then dehydrated with ethanol gradient: 50%, 70%, 80%, and 90% ethanol for 1 hour each. 100% ethanol for 1 hour, repeated once, and then 50% xylene for 30 minutes, repeated once. After dehydration, the tissue was immersed in paraffin for 4 hours and embedded. A paraffin tissue slicer was used to obtain 5 µm tissue sections.

### 5.5.1 Nissl staining

After heating at 60°C for 30 min, 5 µm paraffin sections were dewaxed by soaking in 100% xylene, 50% xylene, and 100% alcohol for 10 min each, and 75% ethanol, 50% ethanol, 30% ethanol, and deionized water for 5 min each. Then, according to the Nissl staining instructions, cresyl violet solution was drop added to the sections, heated at 56 °C for 1 h, differentiated at room temperature for 1-3 min until the background was almost colorless, decolored with gradient alcohol (70%, 95%, and 100%; 20 seconds each), and soaked in xylene. After sealing with neutral resin, data were collected and analyzed under microscope. The results showed that 2.5 nM mAβo*3F and 600 nM mAβ*6E10 caused a significant decrease in the number of neurons in the CA1 and DG regions of the mouse brain, while 2.5 nM mAβ*6E10, 2.5 nM mAβ-ID or 600 nM mAβ-ID had no significant effect on the number of neurons (Figure 5, panels E, F, G).

### Example 6 The Aβo*3F activates glial cells in the mouse brain to produce neuroinflammation

### 6.1 Experimental Materials and Methods

### 6.1.1 Experimental Materials

Anti-GFAP antibody: Cell Signaling Technology, #3670
Anti-Iba-1 antibody: GeneTex, #GTX101495
BCA kit: Thermo Fisher Scientific
RIPA strong lysis buffer: Beijing Solarbio Science & Technology Co.,Ltd.
Protease inhibitors: Millipore
Phosphatase inhibitor (100x): Beijing Solarbio Science & Technology Co.,Ltd.
Mouse IL-1β Detection Kit: Biolegend
Mouse IL-6 Detection Kit: Biolegend

### 6.1.2 Experimental instruments

MD-M5 microplate reader: Molecular Devices, USA
Tissue Lyser II Tissue homogenizer: Qiagen

### 6.1.3 Immunohistochemistry

The dewaxed 5 µm paraffin sections were immersed in citric acid antigen retrieval solution (3 g trisodium citrate, and 0.4 g citric acid dissolved in deionized water, made up to 1 L), boiled in a water bath for 15 min, and cooled naturally at room temperature. After antigen retrieval, the sections were washed 3 times with PBS for 5 min each time, and then fixed with 80% (vol/vol) methanol containing 0.3% H₂O₂ to remove endogenous catalase. Then, the sections were washed 3 times with PBS for 5 min each time, placed in 0.3% Triton X-100 penetration solution, penetrated at room temperature for 20 min, and washed 3 times with PBS for 5 min each time. After the sections were blocked with 10% donkey serum at room temperature for 1 h, the corresponding primary antibody was added and incubated overnight at 4 °C. The next day, the sections were washed 3 times with PBS for 5 min each time, and the corresponding secondary antibody was added and incubated at room temperature for 1 h, and then washed 3 times with PBS for development and observation.

### 6.2 The Aβo*3F can activate microglia and astrocytes

By staining the microglial marker Iba-1 in the hippocampus, it was found that 2.5 nM mAβo*3F and 600 nM mAβ*6E10 can significantly activate microglia in the DG and CA1 regions of hippocampus and increase the expression level of Iba-1 in microglia, but 2.5 nM mAβ*6E10 or mAβ-ID treatment had no significant effect on mice (Figure 6, panels A, C, and D). Moreover, 2.5 nM mAβo*3F and 600 nM mAβ*6E10 can significantly induce microglia in the DG region to enter an ameba-like state with enlarged cell bodies and fewer branches, which represents an over-activated microglia state (Figure 6, panel A). By staining the astrocyte marker GFAP in the mouse hippocampus, it was found that the injection of 2.5 nM mAβo*3F and 600 nM mAβ*6E10 into the mouse brain activated astrocytes in the DG and CA1 regions of hippocampus and increased the expression level of GFAP in hippocampus (Figure 6, panels B, E, and F).

### 6.3 The Aβo*3F increases the level of inflammatory factors in the hippocampus of mice

According to the manufacturer's instructions, IL-6 and IL-1β ELISA kits were used to detect the level of inflammatory factors (IL-6 and IL-1β) in the mouse brain. Briefly, the brain homogenate was diluted and added to the ELISA plate, which was pre-coated with the corresponding capture antibody, and then the corresponding detection antibody and secondary antibody were added. TMB was used as a substrate and the absorbance was measured at 450 nm using an MD-M5 microplate reader. The results showed that the level of IL-1β and IL-6 in the hippocampus were significantly increased in mice injected with 2.5 nM mAβo*3F, 600 nM mAβ*6E10, and 600 nM mAβ-ID, while the level of IL-1β and IL-6 in the brain of mice injected with 2.5 nM mAβ*6E10 or 2.5 nM mAβ-ID was not significantly different from those in the control group (Figure 6, panels G and H).

### Example 7 Detection of the Aβo*3F levels in CSF and plasma of AD patients (MSD method)

### 7.1 Experimental Materials and Methods

### 7.1.1 Experimental Materials

Aβ multifactor detection kit (4G8): Meso Scale Diagnostics

### 7.1.2 Experimental Instruments

MSD-S600 Electrochemiluminescence Detector: Meso Scale Diagnostics, USA

### 7.2 Detection of the Aβo*3F levels in plasma and CSF of AD patients

To evaluate the relevance of Aβos specifically recognized by the antibody 3F (Aβo*3F) and the pathogenesis of AD patients, the level of Aβ42 and Aβ40 contained in Aβo*3F isolated from CSF and plasma of AD patients was tested.

The human plasma and CSF samples used in this study were approved by the Ethics Review Committee of the First Affiliated Hospital of Zhengzhou University, and all the subjects have signed written informed consent before participating in the study. The basic information of the subject samples used in this study was as follows: plasma samples were collected from 20 patients with mild cognitive impairment (MCI) with an average age of 73.3 years (age range, 57-84 years), 20 AD patients with an average age of 69.4 years (age range, 52-85 years), and 20 healthy people with an average age of 70.9 years (age range, 65-78 years). In addition, CSF samples were collected from 7 AD patients with an average age of 68 years (age range, 59-77 years) and 7 non-demented subjects with an average age of 66.3 years (age range, 48-79 years).

To extract the Aβo*3F from plasma and CSF of AD patients and healthy elderly people, human plasma or CSF samples were first added to 100 µL Protein A/G-agarose gel and incubated at 4 °C for 1 h to remove endogenous IgG. The samples were then incubated with Protein A magnetic beads cross-linked with the antibody 3F at 4 °C at room temperature for 2 h. The next day, the beads were washed three times with 0.1% PBST, eluted twice with 20 mM glycine (pH 2.0) for 3 min, and the eluent was neutralized to pH 7 with 1 M Tris. The concentration of the Aβo*3F extracted by immunoprecipitation was detected using the MSD Aβ multifactor detection kit (4G8). The results showed that compared with healthy elderly people, the level of Aβ42 and Aβ40 contained in the Aβo*3F in the CSF and plasma of AD patients was significantly increased (Figure 7). In addition, the level of Aβ42 and Aβ40 contained in the Aβo*3F in the plasma of MCI patients was also significantly higher than those in the control group, and both gradually increased with the development of AD pathology (Figure 7, panels C and D). These results showed that the level of the Aβo*3F in the CSF and plasma of AD patients and AD-derived MCI patients was significantly higher than those in healthy elderly people, and was closely related to the development trend of AD pathology, indicating that the Aβo*3F can be used as a biomarker for AD diagnosis, especially for early diagnosis of AD. The statistical method of one-way ANOVA with Tukey's multiple comparison test in GraphPad Prism 8 software was used for statistics, with 95% confidence interval, *represents P < 0.05, indicating a significant statistical difference; ** represents P < 0.01, indicating a very significant statistical difference; *** represents P < 0.001, indicating an extremely significant statistical difference; **** represents P < 0.0001, indicating an extremely significant statistical difference.

### Example 8 The vaccine prepared using the Aβo*3F as immunogen (ABW vaccine) can significantly improve the cognitive function of AD mice and reduce pathological changes in the mouse brain.

### 8.1 Mouse immunity and antibody titer determination

4-6-week-old female BalB/c mice were selected, 6 mice per group. The Aβo*3F was mixed with Alum adjuvant (ThermoFisher, 77161), and the ratio of Alum adjuvant to antigen was 1: 1 to 1:3 (v/v). Each mouse was subcutaneously inoculated with 100 µg the Aβo*3F, and each immunization was separated by two weeks. Blood was collected from the vein before immunization, and serum was collected and stored at -80 °C. Blood was collected from the vein two weeks after the third immunization and two weeks after the fourth immunization, and serum antibody titers were detected by ELISA.

A 96-well plate was coated with the Aβo*3F (0.5µg/100uL) at 4 °C overnight. After coating, the coating solution was discarded and the plate was washed 3 times with 0.05% PBST. 3% BSA was added into the plate, 300 µL/well, and blocked at 37 °C for 2h. The plate was washed twice with 0.05% PBST. Serum (3% BSA dilution) was added to the plate, 100 µL/well, and incubated at 37°C for 2h. The plate was washed 8 times with 0.05% PBST. HRP-labeled anti-IgG secondary antibody was added to the plate, 100 µL/well, and incubated at 37 °C for 1h. The plate was washed 6 times with PBST. TMB substrate chromogenic solution was added to the plate, 100 µL per well, and the plate was developed at room temperature for 10 min. 0.1 M H₂SO₄ was added to terminate the reaction, 100 µL per well. The absorbance at 450 nm was detected by plate reader.

The results showed that after three times of immunization, the anti-Aβo*3F antibody titer increased significantly, indicating that the Aβo*3F can immune and stimulate the production of anti-Aβo*3F specific antibodies (Figure 8).

### 8.2 ABW vaccine can significantly improve the cognitive ability of AD mice and reduce the pathological changes in the brain of mice.

### 8.2.1 Water maze test

The water maze composed of a pool with a diameter of 110 cm and a platform with a diameter of 10 cm. The pool contained opaque water (22±1°C) and different symbols were marked around the pool to help mice for positioning and identification. During the 5 consecutive days of training, the platform was placed about 1 cm under the water. Mice were placed in the pool from random positions and allowed to swim for 60s to find the platform and stay on the platform for 10s. When the mouse found the platform, the experiment ended and the time to find the platform was recorded. Mice that could not find the platform were guided to the platform and the latency was recorded as 60s. After each training finished, the mice were wiped dry with a dry towel, dried with a heater, and put back to the cage. Training was performed twice a day with an interval of 3-4h. The latency, swimming distance, average swimming speed and exploration pattern of each group of mice were recorded. 24 h after the last learning trial, the platform was removed, the mice were placed in the water from a random site, and let them swim for 60s, and the memory retention ability of the mice was tested in the absence of the platform. The maze was monitored and recorded by a camera installed above it, and parameters such as the time spent in the target quadrant, swimming distance, average swimming speed, exploration path, and number of platform crossings were determined by software analysis.

The above vaccine was injected into 6-month-old AD transgenic mice APP/PS1 for active immunotherapy, and conducted behavioral testing two weeks after 4 times of immunization. The water maze test showed that the vaccine can improve the spatial cognitive ability of AD transgenic mice (Figure 9). During the training stage, compared with AD mice in the PBS control group, the time for AD mice treated with ABW vaccine to reach the platform was significantly shortened (Figure 9, panel A). In the test of removing the platform, AD mice in the ABW vaccine treatment group showed obvious spatial directional swimming behavior. Compared with AD mice in the PBS control group, the time required to reach the platform position was significantly shortened (Figure 9, panel B), the number crossing platform was increased (Figure 9, panel C), and the time spent in the target quadrant was longer (Figure 9, panel D).

### 8.2.2 Determination of Aβ levels in brain homogenates

Preparation of brain homogenate: The right hemibrain of mice was lysed and homogenized in RIPA buffer containing protease inhibitors, and the tissue was centrifuged at 14,000 g for 30 min at 4 °C to collect the supernatant containing soluble Aβ (RIPA soluble fraction). The insoluble precipitate was resuspended in guanidine hydrochloride buffer (5.0 M guanidine hydrochloride, pH 8.0) and centrifuged at 14,000 g for 1 h at 4 °C to obtain the supernatant containing insoluble Aβ (guanidine soluble fraction). Aβ40 ELISA test kit and Aβ42 ELISA test kit from IBL company were used for detection.

The results showed that ABW vaccine treatment could significantly reduce the levels of soluble and insoluble Aβ40 and Aβ42 in the brain of AD mice (Figure 10, panels A-C).

### 8.2.3 Immunostaining of Aβ in mouse brain

In order to explore the effect of ABW vaccine treatment on Aβ plaques in the brain of AD mice, the present invention used immunohistochemistry to detect the level of 6E10-positive Aβ plaques in the brain of AD mice. The results showed that compared with AD mice in the PBS control group, the Aβ plaque staining area in the brain of mice in the ABW vaccine treatment group was significantly reduced (Figure 11).

### Example 9 The single-chain antibody 7B that specifically binds to the Aβo*3F obtained based on phage screening technology can significantly improve the cognitive ability of AD mice and reduce the pathological changes in the mouse brain.

### 9.1 Screening and affinity determination of single-chain antibody 7B that specifically binds to the Aβo*3F obtained based on phage screening technology.

According to the phage screening technology described in patent CN101463082A, the Aβo*3F was diluted to 10-100 µg/mL with coating buffer (PBS, pH=7.4), 4 mL of diluent was added to the immunotube, and coated overnight at 4 °C. After four rounds of enrichment screening, positive clones were screened and identified by sequencing. The sequence of the single-chain antibody was as set forth in SEQ ID NO: 24 (the light and heavy chain CDR sequences were as set forth in SEQ ID NOs: 25-30, respectively), and was named as the antibody 7B. ELISA analysis showed that the antibody had a high affinity with Aβo*3F (Figure 12).

### 9.2 The antibody 7B can significantly improve the cognitive ability of AD mice and reduce the pathological changes in the mouse brain.

In the present invention, APP/PS1 transgenic mice were intranasally administered with the antibody 7B and PBS solvent control, once a day, and the cognitive ability of the mice was detected after 21 days through novel object recognition test. Thioflavin S (THS) staining was used to detect the level of senile plaques in the brain of APP/PS1 transgenic mice.

The experimental method of novel object recognition in mice is as described in item 5.4.1 above. The results showed that the cognitive ability of APP/PS1 mice in the PBS solvent control group for new things was lower than that of the mice in the WT group. Compared with the APP/PS1 mice in the PBS group, the cognitive ability of APP/PS1 mice in the antibody 7B treatment group was significantly improved (Figure 13).

Thioflavin S (THS) staining was applied to mouse brain sections, and the results showed that the antibody 7B treatment can significantly reduce the level of senile plaques in the cortex and hippocampus of APP/PS1 transgenic mice (Figure 14).

### Example 10 Binding of optimized antibody 3F to the Aβo*3F oligomers

The amino acid K at position 100 of the antibody 3F was mutated to R (the position 98 in the antibody numbering when not considering the MA residues introduced for expression as a single-chain antibody, and the variant is named as K98R mutant, and the sequence thereof is shown in SEQ ID NO: 31), the binding of K98R mutant to the Aβo*3F oligomer was tested. The method used was as follows.

The above Aβ oligomers were coated onto a 96-well plate at 100 ng/well and coated overnight at 4 °C. The next day, 3% BSA was used to block the plate for 2 hours at room temperature, washed twice with PBST, and patted dry for use. K98R mutant and the antibody 3F were diluted, starting from 1 µg/ml and 2x dilution for 14 gradients. The diluted antibody solution was added to the ELISA plate coated with the above Aβ oligomers, and parallel replicate wells were made. The plate was incubated at 37 °C for 1 hour, washed 3 times with PBST, and patted dry. 1:10,000 diluted goat anti-human IgG-HRP was added to the plate, incubated at 37 °C for 45 minutes, washed 3 times with PBST, and patted dry. TMB chromogenic solution was added for color development for about 15 minutes. After the reaction was terminated with the stop solution, the OD was read at OD450 nm, and the values were analyzed and compared using graphpad and processed for graphing.

The results were shown in Figure 15. The experiment showed that the binding affinity of the K98R mutant to the Aβo*3F oligomer was further improved, indicating that the combination of this mutant with the Aβo*3F oligomers may be more effective in the treatment of neurodegenerative diseases. The inventors considered the reason for this result and found that when numbering according to IMGT numbering, the amino acid at position 98 is also the residue of the heavy chain CDR3 of the antibody 3F (according to IMGT numbering, the heavy chain/light chain CDR sequences of the antibody 3F are: CDR-H1: GFTFSSYA (SEQ ID NO: 32); CDR-H2: ISNLGLTT (SEQ ID NO: 33); CDR-H3: AKTTSRFDY (SEQ ID NO: 34); CDR-L1: QSISSY (SEQ ID NO: 35); CDR-L2: KAS; CDR-L3: QNSAVRPVT (SEQ ID NO: 36)). The inventors also mutated several other variant sequences to K98R, and the results were similar (data not shown).

### Equivalent solution

Although multiple embodiments of the present invention have been described and illustrated herein, a person of ordinary skill in the art would readily envision various other means and/or structures for achieving the functions described herein and/or obtaining the results and/or one or more advantages described herein, and it is believed that each such change and/or modification is within the scope of the present invention. More broadly, it will be readily understood by those skilled in the art that all the parameters, materials and settings described herein are intended to be exemplary, and that actual parameters, materials and/or settings will depend on the specific application in which the teachings of the present invention are used. Those skilled in the art would recognize or be able to determine many equivalents of the specific embodiments of the present invention described herein using only routine experiments. Therefore, it should be understood that the foregoing embodiments and examples are presented only by way of example, and within the scope of the appended claims and their equivalents, the present invention may be implemented in a manner different from that specifically described and claimed. Any combination of two or more such features, systems, objects, materials and/or methods is included within the scope of the present invention if such features, systems, objects, materials and/or methods are not mutually conflicting.

The phrase "and/or" used in the present specification and claims should be understood to mean "either or both" of the elements so combined, i.e., elements that are present in combination in some cases and not in combination in other cases. In addition to the elements specifically identified by the "and/or" clause, other elements may optionally be present, whether related or unrelated to those specifically identified elements, unless otherwise expressly indicated. Thus, as a non-limiting example, when used in combination with open language such as "comprising," a reference to "A and/or B" may refer to A without B (optionally including other elements in addition to B) in one embodiment; to B without A (optionally including elements in addition to A) in another embodiment; to both A and B (optionally including other elements) in yet another embodiment; and so on.

As used herein in the specification and claims, "or" shall be understood to have the same meaning as "and/or" as defined above. For example, when separating items in a list, "or" or "and/or" shall be understood to be inclusive, i.e., including at least one of a plurality of elements or list of elements, but also including more than one, and optionally, other unlisted items. Only when the contrary term is clearly indicated, such as "only one of" or "exactly one of", or when used in a claim, "consisting of" will refer to comprising exactly one element of a plurality of elements or lists of elements. Generally, when preceded by an exclusive term, such as "either", "one of", "only one of" or "exactly one of", the term "or" used herein should be understood only to represent an exclusive alternative (i.e., "one or the other but not both"). "Substantially consisting of" when used in a claim shall have its ordinary meaning in the field of patent law.

As used herein in the specification and claims, when referring to a list of one or more elements, the phrase "at least one" should be understood to mean at least one element selected from any one or more elements of the list of elements, but does not necessarily include at least one of each element specifically listed in the list of elements, and does not exclude any combination of elements in the list of elements. This definition also allows that any element other than the elements specifically identified in the list of elements referred to by the phrase "at least one" may optionally be present, whether related or unrelated to those specifically identified elements. Thus, as a non-limiting example, in one embodiment, "at least one of A and B" (or equivalently, "at least one of A or B", or equivalently, "at least one of A and/or B") may refer to at least one, optionally including more than one A, without B (and optionally including elements other than B); in another embodiment, to at least one, optionally including more than one B, without A (and optionally including elements other than A); in yet another embodiment, to at least one, optionally including more than one A, and at least one, optionally including more than one B (and optionally including other elements); and so on.

In the claims and the specification, all the conjunctions, such as "comprise", "include", "with", "having", "containing", "involving", "owning", etc., are understood to be open-ended, i.e., meaning including but not limited to. Only the conjunctions "consisting of" and "consisting essentially of" shall be closed or semi-closed conjunctions, respectively.

The use of ordinal terms in the claims, such as "first," "second," "third," etc. to modify claim elements, does not imply any priority, precedence, or order of one claim element relative to another claim element, or a temporal order of actions in a method, but merely serves as a label to distinguish one claim element with a certain name from another element with the same name (but for ordinal terms) to distinguish claim elements.

## Claims

1. An isolated Aβo*3F, wherein the Aβo*3F is an Aβ oligomer specifically bound by the antibody 3F or variant thereof, wherein total molecular weight of the isolated Aβo*3F is about 588 kDa based on size exclusion chromatography (SEC) analysis, the light and heavy chain CDR sequences of the antibody 3F are as set forth in SEQ ID NOs: 17-22, respectively, and the variant is K98R mutant or the antibody 7B, of which the light and heavy chain CDR sequences are as set forth in SEQ ID NOs: 25-30 or SEQ ID NOs: 32-35 and KAS, respectively.

2. The Aβo*3F according to claim 1, which is prepared *in vitro.*

3. A composition comprising the Aβo*3F according to claim 1 or 2.

4. The composition according to claim 3, which is an immunogenic composition, a pharmaceutical composition or a vaccine.

5. The composition according to claim 3 or 4, further comprising a pharmaceutically acceptable excipient and/or adjuvant.

6. The composition according to any one of claims 3-5, further comprising additional active ingredients, preferably, the additional active ingredients are other forms of Aβ oligomers, prefibrils and mature fibers, such as Aβ dimers, trimers, hexamers, dodecamers, ASPD, ADDL, etc., preferably, the additional active ingredients are binding agents that specifically bind to the Aβo*3F, preferably, the binding agents that specifically bind to the Aβo*3F are the antibodies 3F, K98R and 7B, more preferably, the binding agents that specifically bind to the Aβo*3F are in a separated state from the Aβo*3F for simultaneous or sequential administration.

7. An *in vitro* method for preparing the Aβo*3F according to claim 1 or 2, comprising the following steps: dissolving Aβ42, Aβ40 or other forms of Aβ in 50 mM NaOH to a concentration of 1 mg/mL, vortexing for 3-5 min, sonicating for 1 min, and then diluting to 10 µM with pre-cooled PBS; centrifuging at 21,000 g for 30-40 min at 4°C, discarding the precipitate (approximately 5% of the starting volume ) to obtain Aβ monomers; incubating the Aβ monomers statically at 25°C for 2 days, and incubating them with Protein A magnetic beads cross-linked with the antibody 3F at 4°C overnight; on the next day, washing the magnetic beads three times with 0.1% PBST, eluting with 20-100 mM glycine (pH 2.0) for 3-5 min, eluting twice, and neutralizing the eluate to pH 7 with 1 M Tris to obtain the Aβo*3F.

8. Use of Aβo*3F as a target in the manufacture of a medicament for preventing and/or treating MCI and/or AD in a subject, wherein the Aβo*3F is an Aβ oligomer specifically bound by the antibody 3F or variant thereof, wherein total molecular weight of the Aβo*3F is about 588 kDa based on size exclusion chromatography (SEC) analysis, the light and heavy chain CDR sequences of the antibody 3F are as set forth in SEQ ID NOs: 17-22, respectively, and the variant is K98R mutant or the antibody 7B, of which the light and heavy chain CDR sequences are as set forth in SEQ ID NOs: 25-30 or SEQ ID NOs: 32-35 and KAS, respectively.

9. The use according to claim 8, wherein the medicament is an antiserum, an antibody, or a small molecule drug that specifically binds to the Aβo*3F, preferably, the antibody is a polyclonal antibody or a monoclonal antibody or an antigen-binding fragment thereof.

10. A binding agent that specifically binds to the Aβo*3F according to claim 1 or 2, wherein the binding agent is the antibodies 3F, K98R and 7B.

11. The binding agent according to claim 10, wherein the binding agent is an antiserum, an antibody, or a small molecule drug that specifically binds to Aβo*3F, preferably, the antibody is a polyclonal antibody or a monoclonal antibody or an antigen-binding fragment thereof.

12. A method for preparing the binding agent according to claim 10 or 11, comprising the following steps: immunizing an animal with an immunologically effective amount of the Aβo*3F according to claim 1 or 2, or performing screening in a phage display peptide library, an antibody library and a compound library using the Aβo*3F according to claim 1 or 2 as a substrate.

13. The method according to claim 12, wherein the animal is a mouse, a rat, a guinea pig, a rabbit, a sheep, a goat, a monkey, a horse, a cow, an alpaca, or a non-human primate.

14. A composition comprising the binding agent according to claim 10 or 11 or the binding agent obtained by the method according to claim 12 or 13, preferably, the composition further comprises the Aβo*3F according to claim 1 or 2, more preferably, the binding agent that specifically binds to the Aβo*3F and the Aβo*3F are in a separated state for simultaneous or sequential administration.

15. Use of the Aβo*3F according to claim 1 or 2, the composition according to any one of claims 3-6 and 14-15, or the binding agent according to claim 10 or 11 in the manufacture of a medicament for preventing and/or treating MCI and/or AD in a subject.

16. The Aβo*3F according to claim 1 or 2, the composition according to any one of claims 3-6 and 14-15, or the binding agent according to claim 10 or 11, for use as a medicament for preventing and/or treating MCI and/or AD in a subject.

17. A method for preventing and/or treating MCI and/or AD in a subject, comprising administering to the subject a preventively and/or therapeutically effective amount of the Aβo*3F according to claim 1 or 2, the composition according to any one of claims 3-6 and 14-15, or the binding agent according to claim 10 or 11.

18. Use of Aβo*3F as a diagnostic target for MCI and/or AD in a subject, wherein the Aβo*3F is an Aβ oligomer in the subject specifically bound by the antibody 3F or variant thereof, wherein total molecular weight of the Aβo*3F is approximately 588 kDa based on size exclusion chromatography (SEC) analysis, and the light and heavy chain CDR sequences of the antibody 3F are as set forth in SEQ ID NOs: 17-22, respectively, the variant is K98R mutant or the antibody 7B, of which the light and heavy chain CDR sequences are as set forth in SEQ ID NOs: 25-30 or SEQ ID NOs: 32-35 and KAS, respectively.

19. Use of Aβo*3F as a preventive and/or therapeutic target for MCI and/or AD in a subject, wherein the Aβo*3F is an Aβ oligomer in the subject specifically bound by the antibody 3F or variant thereof, wherein total molecular weight of the Aβo*3F is approximately 588 kDa based on size exclusion chromatography (SEC) analysis, and the light and heavy chain CDR sequences of the antibody 3F are as set forth in SEQ ID NOs: 17-22, respectively, the variant is K98R mutant or the antibody 7B, of which the light and heavy chain CDR sequences are as set forth in SEQ ID NOs: 25-30 or SEQ ID NOs: 32-35 and KAS, respectively.

20. The use according to claim 15, the Aβo*3F, the composition or the binding agent according to claim 16, the method according to claim 17 or the use according to claim 18 or 19, wherein the subject is a human, a non-human primate, a cat or a dog.
